(19) Europäisches Patentamt
European Patent Office
Office européen des brevets

(11) Veröffentlichungsnummer: **0 055 490**
**A1**

(12) # EUROPÄISCHE PATENTANMELDUNG

(21) Anmeldenummer: 81110829.9

(22) Anmeldetag: 29.12.81

(51) Int. Cl.$^3$: C 07 D 273/04
C 07 D 413/04, A 61 K 31/535

(30) Priorität: 31.12.80 HU 316380
31.12.80 HU 316480

(43) Veröffentlichungstag der Anmeldung:
07.07.82 Patentblatt 82/27

(84) Benannte Vertragsstaaten:
BE CH DE FR GB IT LI NL SE

(71) Anmelder: **RICHTER GEDEON VEGYESZETI GYAR RT**
Gyömröi ut 21
H-1103 Budapest(HU)

(72) Erfinder: **Kisfaludy, Lajos, Dr. Dipl.-Chem. Ing.**
Riado u. 6/a
H-1026 Budapest(HU)

(72) Erfinder: **Ürögdi, Lászlo, Dipl.-Chem. Ing.**
Névtelen u. 35
H-1163 Budapest(HU)

(72) Erfinder: **Patthy, Agnes, geb. Lukáts**
Fenyö u. 10
H-1016 Budapest(HU)

(72) Erfinder: **Dancsi, Lajos**
Szendrö u. 18
H-1126 Budapest(HU)

(72) Erfinder: **Szilbereky, Jenö, Dipl.-Chem. Ing.**
Szamos u. 7
H-1122 Budapest(HU)

(72) Erfinder: **Moravcsik, Ernö**
Lágymányosi u. 28
H-1111 Budapest(HU)

(72) Erfinder: **Tüdös, Helga, geb. Fauer, Dr. Chem.**
Felhö u. 18
H-1125 Budapest(HU)

(72) Erfinder: **Ötvös, László, Dr. Chem.**
Boróka u. 13
H-1025 Budapest(HU)

(72) Erfinder: **Tegyei, Zsuzsanna, Dr. Chem.**
Györi u. 2/a
H-1123 Budapest(HU)

(72) Erfinder: **Pálosi, Eva, Dr.**
Vend u. 21
H-1025 Budapest(HU)

(72) Erfinder: **Sarkadi, Adám, Dr.**
Thököly u. 85
H-1143 Budapest(HU)

(72) Erfinder: **Szporny, Lászlo, Dr.**
Szabolcska M. u. 7
H-1114 Budapest(HU)

(74) Vertreter: **Beszédes, Stephan G. Dr.**
Münchener Strasse 80a Postfach 1168
D-8060 Dachau(DE)

(54) **Tetrahydro-1,2,4-oxadiazin-5-onderivate, Verfahren zu ihrer Herstellung und diese Verbindungen enthaltende Arzneimittel.**

(57) Tetrahydro-1,2,4-oxadiazin-5-on-derivate der allgemeinen Formel

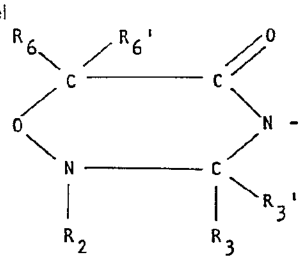

I ,

sowie Verfahren zu ihrer Herstellung.

Diese Verbindungen können in Arzneimitteln auf dem Gebiet der Therapie, insbesondere gegen Erkrankungen des Zentralnervensystemes, verwendet werden.

EP 0 055 490 A1

Croydon Printing Company Ltd.

Die Erfindung betrifft neue Tetrahydro-1,2,4-oxadiazin-5-onderivate, ein Verfahren zu ihrer Herstellung und diese Verbindungen enthaltende Arzneimittel, insbesondere solche mit Wirkungen auf das Zentralnervensystem.

Das Tetrahydro-1,2,4-oxadiazin und einige substituierte Derivate desselben wurden erst in den letzteren Jahren bekannt. Ihre Synthese wurde zuerst von Calgano und Mitarbeitern (J. Org. Chem. 39 [1974], 162) beschrieben; von diesen Verfassern wurden durch die Cycloaddition von 1-Aroylaziridinen an Nitrone die entsprechenden 4-Aroyltetrahydro-1,2,4-oxadiazine erhalten. Später wurden von F. G. Riddel und Mitarbeitern (Heterocycles 9 [1978], 267; Tetrahedron 35 [1979], 1 391) durch Kondensation von N-(Alkyl)-O-(methylaminoäthyl)-hydroxylaminen mit Formaldehyd Tetrahydro-1,2,4-oxadiazine hergestellt. Über eine etwaige biologische Wirksamkeit dieser Produkte ist jedoch im Schrifttum nichts erwähnt und es wurden bisher auch keine in der 5-Stellung eine Oxogruppe aufweisenden Tetrahydro-1,2,4-oxadiazinderivate beschrieben.

Der Erfindung liegt die Aufgabe zugrunde, überlegene pharmakologische Wirkungen aufweisende neue Tetrahydro-1,2,4-oxadiazin-5-onderivate, ein Verfahren zur Herstellung derselben und diese Verbindungen enthaltende Arzneimittel zu schaffen.

Das Obige wurde überraschenderweise durch die Erfindung erreicht.

Es wurde nämlich überraschenderweise festgestellt, daß die im folgenden festgelegten substituierten Tetrahydro-1,2,4-oxadiazin-5-one überlegene wertvolle pharmakologische

Eigenschaften, insbesondere vorteilhafte Wirkungen auf das Zentralnervensystem, haben.

Gegenstand der Erfindung sind daher Tetrahydro-1,2,4-oxadiazin-5-onderivate der allgemeinen Formel

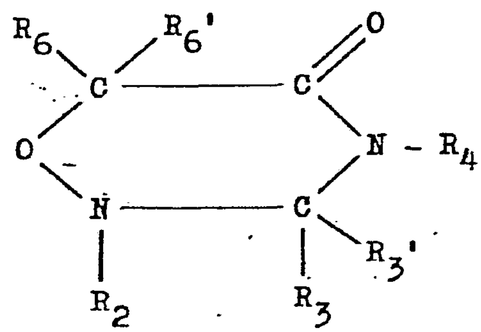

I ,

worin

$R_2$ für Wasserstoff, einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest, einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest oder einen N-(Benzyloxycarbonyl)-glycylrest steht,

| | |
|---|---|
| $R_3$ | einen Alkylrest mit 1 bis 5 Kohlenstoffatom(en), einen, gegebenenfalls durch 1 oder mehr Nitrogruppe(n), Carboxygruppe(n), Hydroxygruppe(n), Dialkylaminorest(e) mit 1 bis 4 Kohlenstoffatom(en) in jedem Alkylteil und/oder Alkoxyrest(e) mit 1 bis 4 Kohlenstoffatom(en) substituierten, Phenylrest, einen Naphthylrest, einen Nitrofurylrest oder einen Thienylrest bedeutet und |
| $R_3'$ | für Wasserstoff steht oder |
| $R_3$ und $R_3'$ zusammen | einen Pentamethylenrest darstellen, |
| $R_4$ | für Wasserstoff oder einen Acetylrest steht, |
| $R_6$ | Wasserstoff, einen Alkylrest mit 1 bis 5 Kohlenstoffatom(en), einen Phenylrest oder einen Benzylrest bedeutet und |
| $R_6'$ | für Wasserstoff steht oder |
| $R_6$ und $R_6'$ | je einen Phenylrest bedeuten, |

sowie ihre optisch aktiven Antipoden und Gemische derselben, im Falle daß $R_6'$ für Wasserstoff steht und $R_6$ einen Alkylrest mit 1 bis 5 Kohlenstoffatom(en), einen Phenylrest oder einen Benzylrest bedeutet.

In den erfindungsgemäßen Tetrahydro-1,2,4-oxadiazin-5-onderivaten der allgemeinen Formel I, bei welchen $R_6'$ für Wasserstoff steht und $R_6$ einen Alkylrest mit 1 bis 5 Kohlenstoffatom(en), einen Phenylrest oder einen Benzylrest bedeutet, hat das Kohlenstoffatom in der 6-Stellung eine

asymmetrische Konfiguration, so daß solche Verbindungen in racemischen oder optisch aktiven Formen existieren können. Die Erfindung umfaßt alle diese Formen.

Vorzugsweise ist beziehungsweise sind das beziehungsweise die Halogenatom(e), durch welches beziehungsweise welche der Alkylcarbonylrest oder der Benzoylrest, für den $R_2$ stehen kann, substituiert sein kann, Chlor und/oder Fluor, insbesondere Chlor.

Es ist auch bevorzugt, daß der Alkylcarbonylrest, für den $R_2$ stehen kann, ein solcher mit 1 bis 4, insbesondere 1 oder 2, ganz besonders 1, Kohlenstoffatom(en) im Alkylteil ist.

Ferner ist es bevorzugt, daß der beziehungsweise die Alkylrest(e), durch welchen beziehungsweise welche der Carbamoylrest, für den $R_2$ stehen kann, N-substituiert sein kann, ein solcher beziehungsweise solche mit 1 bis 4, insbesondere 3 oder 4, Kohlenstoffatom(en) ist beziehungsweise sind. Dabei sind der n-Propylrest und der n-Butylrest ganz besonders bevorzugt. Sofern der Carbamoylrest, für den $R_2$ stehen kann, N-substituiert ist, ist es bevorzugt, daß er nur 1 Substituenten aufweist.

Weiterhin ist es bevorzugt, daß der beziehungsweise die Dialkylaminoalkylrest(e), durch welchen beziehungsweise welche der Carbamoylrest, für den $R_2$ stehen kann, N-substituiert sein kann, ein solcher beziehungsweise solche mit 1 oder 2, insbesondere 2, Kohlenstoffatom(en) in jedem Alkylteil ist beziehungsweise sind.

Vorzugsweise ist der Alkylrest, für den $R_3$ stehen kann, ein solcher mit 1 bis 4, insbesondere 2 oder 3, ganz besonders 3, Kohlenstoffatom(en). Dabei ist der n-Propylrest ganz besonders bevorzugt.

Es ist auch bevorzugt, daß der beziehungsweise die Dialkylaminorest(e), durch welchen beziehungsweise welche der Phenylrest, für den $R_3$ stehen kann, substituiert sein kann, ein solcher beziehungsweise solche mit 1 oder 2, insbesondere 1, Kohlenstoffatom(en) in jedem Alkylteil ist beziehungsweise sind.

Ferner ist es bevorzugt, daß der beziehungsweise die Alkoxyrest(e), durch welchen beziehungsweise welche der Phenylrest, für den $R_3$ stehen kann, substituiert sein kann, ein solcher mit 1 oder 2, insbesondere 1, Kohlenstoffatom(en) ist beziehungsweise sind.

Weiterhin ist es bevorzugt, daß der Naphthylrest, für den $R_3$ stehen kann, ein $\alpha$-Naphthylrest ist.

Vorzugsweise ist der Nitrofurylrest, für den $R_3$ stehen kann, ein 5-Nitrofurylrest, insbesondere 5-Nitrofur-2-ylrest.

Es ist auch bevorzugt, daß der Thienylrest, für den $R_3$ stehen kann, ein Thien-2-ylrest ist.

Ferner ist es bevorzugt, daß der Alkylrest, für den $R_6$ stehen kann, ein solcher mit 1 oder 2, insbesondere 1, Kohlenstoffatom(en) ist.

Weiterhin ist es bevorzugt, daß der beziehungsweise die Alkoxyrest(e), durch welchen beziehungsweise welche der Benzoylrest, für den $R_2$ stehen kann, substituiert sein kann, ein Methoxyrest beziehungsweise Methoxyreste ist beziehungsweise sind.

Besonders bevorzugte erfindungsgemäße Verbindungen sind 2-Acetyl-3-phenyltetrahydro-1,2,4-oxadiazin-5-on, 2-Acetyl-3-phenyl-6-methyltetrahydro-1,2,4-oxadiazin-5-on und seine optisch aktiven Antipoden, 2,4-Diacetyl-3-phenyltetrahydro-1,3,4-oxadiazin-5-on, 2-Acetyl-3-(4'-fluorphenyl)-tetrahydro-1,2,4-oxadiazin-5-on und 2-Benzyloxycarbonyl-3-phenyltetrahydro-1,2,4-oxadiazin-5-on, ganz besonders das erste.

Gegenstand der Erfindung ist auch ein Verfahren zur Herstellung der erfindungsgemäßen Verbindungen, welches dadurch gekennzeichnet ist, daß

a) zur Herstellung von Tetrahydro-1,2,4-oxadiazin-5-onderivaten der allgemeinen Formel I,

bei welchen

$R_2$ von Wasserstoff verschieden ist und

$R_4$ für Wasserstoff steht,

α-Aminooxycarbonsäureamide der allgemeinen Formel

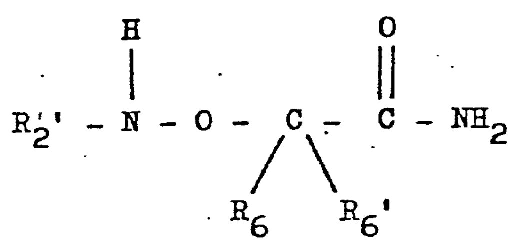

II ,

worin

$R_2'$ für einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest,

einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest oder einen N-(Benzyloxycarbonyl)--glycyclrest steht und

$R_6$ und $R_6'$ wie oben festgelegt sind,

in protonischen (protischen) oder aprotonischen (aprotischen) Medien in Gegenwart von Säuren mit Oxoverbindungen der allgemeinen Formel

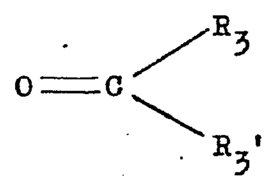

III ,

worin $R_3$ und $R_3'$ wie oben festgelegt sind, kondensiert werden

oder

b) zur Herstellung von Tetrahydro-1,2,4-oxadiazin-5--onderivaten der allgemeinen Formel I, bei welchen $R_2$ und $R_4$ Wasserstoff bedeuten, Tetrahydro-1,2,4--oxadiazin-5-onderivate der allgemeinen Formel I,

bei welchen

$R_2$ für einen Benzyloxycarbonylrest steht und

$R_4$ Wasserstoff bedeutet,

in Gegenwart von Katalysatoren mit Wasserstoff, also mit katalytisch erregtem Wasserstoff, hydriert werden

und/oder

c) zur Herstellung von Tetrahydro-1,2,4-oxadiazin-5--onderivaten der allgemeinen Formel I, bei welchen $R_2$ für einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest, einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest oder einen N-(Benzyloxycarbonyl)-glycylrest steht, Tetrahydro--1,2,4-oxadiazin-5-onderivate der allgemeinen Formel I, bei welchen $R_2$ für Wasserstoff steht, mit Acylhalogeniden beziehungsweise Carbonsäureanhydriden der allgemeinen Formel

$$R_2' - X \qquad IV,$$

worin

$R_2'$ wie oben festgelegt ist und

X für ein Halogenatom oder einen Acyloxyrest der allgemeinen Formel

$$R_2' - O - \qquad V,$$

in welchletzterer

$R_2'$ wie oben festgelegt ist,

steht,

umgesetzt werden

oder

d) zur Herstellung von Tetrahydro-1,2,4-oxadiazin-5--onderivaten der allgemeinen Formel I, bei welchen $R_2$ einen Formylrest bedeutet, Tetrahydro-1,2,4--oxadiazin-5-onderivate der allgemeinen Formel I, bei welchen $R_2$ für Wasserstoff steht, in Gegenwart von Kondensationsmitteln mit Ameisensäure umgesetzt werden

oder

e) zur Herstellung von Tetrahydro-1,2,4-oxadiazin-5--onderivaten der allgemeinen Formel I, bei welchen $R_4$ für einen Acetylrest steht, Tetrahydro-1,2,4--oxadiazin-5-onderivate der allgemeinen Formel I, bei welchen $R_4$ Wasserstoff bedeutet, unter energischen Reaktionsbedingungen mit Acetylchlorid oder Essigsäureanhydrid umgesetzt werden

oder

f) zur Herstellung von Tetrahydro-1,2,4-oxadiazin-5--onderivaten der allgemeinen Formel I, bei welchen $R_2$ für einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest steht, Tetrahydro-1,2,4-oxadiazin--5-onderivate der allgemeinen Formel I, bei welchen $R_2$ einen Phenoxycarbonylrest bedeutet,

mit Ammoniak oder mit passenden primären oder sekundären Aminen umgesetzt werden

oder

g) zur Herstellung von Tetrahydro-1,2,4-oxadiazin-5--onderivaten der allgemeinen Formel I, bei welchen $R_2$ für einen durch einen Alkylrest mit 1 bis 8 Kohlenstoffatom(en) N-substituierten Carbamoylrest steht, Tetrahydro-1,2,4-oxadiazin-5-onderivate der allgemeinen Formel I, bei welchen $R_2$ Wasserstoff bedeutet, mit Alkylisocyanaten der allgemeinen Formel

$$R - N{=}C{=}O \qquad VI ,$$

worin R für einen Alkylrest mit 1 bis 8 Kohlenstoffatom(en) steht, umgesetzt werden.

Als aprotonische Medien für die Umsetzung der Variante a) des erfindungsgemäßen Verfahrens können zweckmäßig organische Lösungsmittel vom, vorzugsweise aromatischen, Kohlenwasserstofftyp, insbesondere Benzol und/oder Toluol, verwendet werden. Zur Erhöhung der Löslichkeit der umzusetzenden Verbindungen können solche Lösungsmittel nach Bedarf mit einem Gehalt an kleineren bis gleichen Mengen von Butylacetat eingesetzt werden. Als die Umsetzung katalysierende Säuren können zum Beispiel Mineralsäuren, wie Schwefelsäure und besonders vorteilhaft Campher-10-sulfonsäure, verwendet werden (im Schrifttum wurde die Verwendung von Campher-10-sulfonsäure für solche Zwecke noch nicht erwähnt). Zur Entfernung des bei der Umsetzung entstehenden Wassers aus dem Reaktionsgemisch wird die Umsetzung zweckmäßig bei der Siedetemperatur des Reaktionsgemisches durchgeführt und das entstandene Wasser mit Hilfe eines entsprechenden Aufsatzes laufend abgetrennt.

Als protonisches Medium für die Umsetzung der Variante a)

des erfindungsgemäßen Verfahrens kann zum Beispiel ein Gemisch von Essigsäure und Essigsäureanhydrid im Volumverhältnis von 1 : 1, welches als Säurezusatz zweckmäßig eine Mineralsäure oder Campher-10-sulfonsäure enthält, verwendet werden. In diesem Fall kann die Umsetzung mit gutem Erfolg bei Raumtemperatur durchgeführt werden.

In beiden Fällen ist es zweckmäßig, den Verlauf der Reaktion durch Dünnschichtchromatographie zu verfolgen. Nach dem Ablauf der Reaktion kann das Lösungsmittel auf die übliche Weise entfernt und das als Rückstand erhaltene Produkt aus geeigneten Lösungsmitteln kristallisiert werden.

Das katalytische Hydrieren der, zweckmäßig nach der Variante a) des erfindungsgemäßen Verfahrens herstellbaren, erfindungsgemäßen Tetrahydro-1,2,4-oxadiazin-5-onderivate der allgemeinen Formel I, bei welchen $R_2$ für einen Benzyloxycarbonylrest steht und $R_4$ Wasserstoff bedeutet, bei welchem der Benzyloxycarbonylrest abgespalten wird, nach der Variante b) des erfindungsgemäßen Verfahrens wird zweckmäßig in organischen Lösungsmitteln oder Lösungsmittelgemischen, zum Beispiel Methanol und/oder Äthylacetat, vorteilhaft unter Normaldruck, durchgeführt. Als Katalysator wird vorteilhaft Palladium/Aktivkohle verwendet. Zur Vermeidung der Bildung von Nebenprodukten ist es empfehlenswert, das Fortschreiten der Hydrierungsreaktion mittels Dünnschichtchromatographie zu kontrollieren; die Reaktion wird abgebrochen, wenn die dünnschichtchromatographische Analyse zeigt, daß keine nicht umgesetzte Ausgangsverbindung mehr im Reaktionsgemisch vorhanden ist, aber noch keine nennenswerten Mengen von Nebenprodukten entstanden sind. Es kann dann der Katalysator abfiltriert, das Filtrat eingedampft und der Rückstand durch Verreiben mit einem indifferenten Lösungsmittel und/oder Umkristallisieren isoliert werden.

Das Umsetzen der, beispielsweise nach der Variante b)

des erfindungsgemäßen Verfahrens herstellbaren, erfindungsgemäßen Tetrahydro-1,2,4-oxadiazin-5-onderivate der allgemeinen Formel I, bei welchen $R_2$ für Wasserstoff steht, mit den den einzuführenden Acylrest aufweisenden Acylhalogeniden beziehungsweise Carbonsäureanhydriden der allgemeinen Formel IV zu den entsprechenden erfindungsgemäßen Tetrahydro-1,2,4--oxadiazin-5-onderivaten der allgemeinen Formel I, bei welchen $R_2$ für einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest, einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest oder einen N-(Benzyloxycarbonyl)-glycylrest steht, wobei keine der Bedeutungen von $R_3$ , $R_3'$ , $R_4$ , $R_6$ und $R_6'$ gegenüber denen in den genannten Ausgangsverbindungen verändert ist, nach der Variante c) des erfindungsgemäßen Verfahrens kann nach bekannten und in der präparativen organischen Chemie üblichen N-Acylierungsverfahrensweisen unter den üblichen Reaktionsbedingungen durchgeführt werden. Zweckmäßig werden Lösungen der genannten erfindungsgemäßen Tetrahydro-1,2,4--oxadiazin-5-onderivatausgangsverbindungen der allgemeinen Formel I in wasserfreien organischen Lösungsmitteln in Gegenwart von säurebindenden Mitteln bei 0°C mit gekühlten Lösungen der entsprechenden Acylhalogenide, vorteilhaft Acylchloride, der allgemeinen Formel IV in organischen Lösungsmitteln versetzt. Bei Verwendung von Carbonsäureanhydriden der allgemeinen Formel IV als Acylierungsmitteln können diese selbst als Reaktionsmedium dienen.

Die bei der Variante c) des erfindungsgemäßen Verfahrens als Ausgangsverbindungen verwendeten erfindungsgemäßen

Tetrahydro-1,2,4-oxadiazin-5-onderivate der allgemeinen Formel I können außer am Stickstoffatom in der 2-Stellung auch am Stickstoffatom in der 4-Stellung ein austauschbares Wasserstoffatom aufweisen. Bei der unter den oben angegebenen Bedingungen durchgeführten Acylierungsreaktion wird immer selektiv das Stickstoffatom in der 2-Stellung acyliert.

Wenn aber die Umsetzung der erfindungsgemäßen Tetrahydro-1,2,4-oxadiazin-5-onderivate der allgemeinen Formel I, bei welchen sowohl $R_2$ als auch $R_4$ Wasserstoff bedeuten, mit Acetylchlorid oder Essigsäureanhydrid unter energischeren Reaktionsbedingungen (mit einem Überschuß des Acylierungsmittels mit längerem Erhitzen zum Sieden) durchgeführt wird, dann können die entsprechenden erfindungsgemäßen Tetrahydro-1,2,4-oxadiazin-5-onderivate der allgemeinen Formel I, bei welchen sowohl $R_2$ als auch $R_4$ für je 1 Acetylrest stehen, das heißt 2,4-Diacetylverbindungen, als Reaktionsprodukte erhalten werden. Ebenso kann in die in der 2-Stellung schon acylierten, aber in der 4-Stellung Wasserstoff aufweisenden erfindungsgemäßen Tetrahydro-1,2,4-oxadiazin-5-onderivate der allgemeinen Formel I ein weiterer Acetylrest in die 4-Stellung eingeführt werden. Diese Umsetzungen beinhaltet die Variante e) des erfindungsgemäßen Verfahrens.

Die Variante b) des erfindungsgemäßen Verfahrens [Abspalten einer Benzyloxycarbonylgruppe in der 2-Stellung] und die Variante c) des erfindungsgemäßen Verfahrens [N-Acylierung] können auch gleichzeitig in einer einzigen Reaktionsstufe durchgeführt werden, wozu das den in die 2-Stellung einzuführenden Acylrest aufweisende Carbonsäureanhydrid schon bei der katalytischen Hydrierung als Reaktionsmedium verwendet wird. In diesem Fall findet beim Abspalten des 2-Benzyloxycarbonylrestes gleichzeitig auch die Acylierung in der 2-Stellung statt.

Die Variante d) des erfindungsgemäßen Verfahrens wird

zweckmäßig in der Weise durchgeführt, daß die erfindungsgemäßgen Tetrahydro-1,2,4-oxadiazin-5-onderivate der allgemeinen Formel I, bei welchen $R_2$ für Wasserstoff steht, in wasserfreien organischen Lösungsmitteln, zum Beispiel in wasserfreiem Tetrahydrofuran, in Gegenwart von Kondensationsmitteln, insbesondere von Dicyclohexylcarbodiimid, mit der Ameisensäure umgesetzt werden. Diese Umsetzung wird zweckmäßig unter Kühlen, zum Beispiel bei 0 bis -10°C, durchgeführt.

Was die Herstellung der erfindungsgemäßen Tetrahydro--1,2,4-oxadiazin-5-onderivate der allgemeinen Formel I, bei welchen $R_2$ für einen Formylrest steht, betrifft, ist die Variante d) des erfindungsgemäßen Verfahrens vorteilhafter als dessen Variante c).

Das Überführen der erfindungsgemäßen Tetrahydro-1,2,4--oxadiazin-5-onderivate der allgemeinen Formel I, bei welchen $R_2$ für einen Phenoxycarbonylrest steht, in die entsprechenden Tetrahydro-1,2,4-oxadiazin-5-onderivate der allgemeinen Formel I, bei welchen $R_2$ einen gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest bedeutet, nach der Variante f) des erfindungsgemäßen Verfahrens kann zweckmäßig in der Weise durchgeführt werden, daß die ersteren in bezüglich der Reaktion inerten organischen Lösungsmitteln, zum Beispiel in Chloroform und/oder Tetrahydrofuran, mit den entsprechenden organischen primären oder sekundären Aminen beziehungsweise zur Herstellung der erfindungsgemäßen Tetrahydro-1,2,4-oxadiazin-5-onderivate der allgemeinen Formel I, bei welchen $R_2$ einen nicht N-substituierten Carbamoylrest bedeutet, das heißt nicht N-substituierten Carbamoylderivate, mit wäßrigen Ammoniumhydroxydlösungen umgesetzt werden. Zweckmäßig wird also die Umsetzung mit organischen Aminen in homogenem Medium und die Umsetzung mit wäßrigem Ammoniak in heterogenem Medium

durchgeführt. Zum Binden des während der Reaktion freiwerdenden Phenoles kann ein Überschuß des als Reaktionsteilnehmer eingesetzten Amines oder ein organisches tertiäres Amin, zum Beispiel Triäthylamin, verwendet werden. Die Umsetzung kann bei Raumtemperatur auch einige Tage in Anspruch nehmen, sie ist aber bei der Siedetemperatur des Reaktionsgemisches in einigen Stunden beendet. Das Reaktionsprodukt kann nach den üblichen präparativen Verfahrensweisen aus dem Reaktionsgemisch isoliert werden.

Die Umsetzung der erfindungsgemäßen Tetrahydro-1,2,4-oxadiazin-5-onderivate der allgemeinen Formel I, bei welchen $R_2$ für Wasserstoff steht, zu den erfindungsgemäßen Tetrahydro-1,2,4-oxadiazin-5-onderivaten der allgemeinen Formel I, bei welchen $R_2$ für einen durch einen Alkylrest mit 1 bis 8 Kohlenstoffatom(en) N-substituierten Carbamoylrest steht, nach der Variante g) des erfindungsgemäßen Verfahrens kann unter den üblichen Bedingungen solcher Acylierungsreaktionen, zweckmäßig in wasserfreien organischen Lösungsmitteln, zum Beispiel in wasserfreiem Tetrahydrofuran, durchgeführt werden. Das Reaktionsprodukt kann ebenfalls nach den üblichen präparativen Verfahrensweisen isoliert werden.

Die erfindungsgemäßen optisch aktiven Tetrahydro-1,2,4-oxadiazin-5-onderivate der allgemeinen Formel I können in der Weise hergestellt werden, daß bei den Umsetzungen der Varianten a) bis g) des erfindungsgemäßen Verfahrens die entsprechenden optisch aktiven Formen der entsprechenden Ausgangsverbindungen, bei welchen $R_6'$ für Wasserstoff steht und $R_6$ einen Alkylrest mit 1 bis 5 Kohlenstoffatom(en), einen Phenylrest oder einen Benzylrest bedeutet, eingesetzt werden.

Die bei der Variante a) des erfindungsgemäßen Verfahrens als Ausgangsstoffe verwendeten $\alpha$-Aminooxycarbonsäureamide der allgemeinen Formel II sind bereits aus dem Schrifttum (zum Beispiel A. Frank und K. Riedl, Mh. Chem. 92 [1961], 725;

Blaser und Mitarbeiter, Helv. Chim. Acta 1969, 569; M. Bellando und Mitarbeiter, Il Farm. Ed. Sci. 1976, 31 [3], 169) bekannte Verbindungen, welche nach den im genannten Schrifttum beschriebenen Verfahren oder durch Umsetzen von Acylhydroxamsäuren mit entsprechenden α-Halogencarbonsäureamiden hergestellt worden sein können.

Ferner sind erfindungsgemäß Arzneimittel, welche 1 oder mehr der erfindungsgemäßen Tetrahydro-1,2,4-oxadiazin-5-onderivate der allgemeinen Formel I als Wirkstoff beziehungsweise Wirkstoffe, gegebenenfalls zusammen mit 1 oder mehr pharmazeutisch üblichen festen oder flüssigen Trägerstoff(en) und/oder Hilfsstoff(en), enthalten, vorgesehen.

Die erfindungsgemäßen Tetrahydro-1,2,4-oxadiazin-5-onderivate haben nämlich wie bereits erwähnt wertvolle pharmakologische Wirkungen, insbesondere auf das Zentralnervensystem.

Die pharmakologischen Wirkungen der erfindungsgemäßen Tetrahydro-1,2,4-oxadiazin-5-onderivate der allgemeinen Formel I wurden in Tierversuchen nach den üblichen pharmakologischen Verfahrensweisen nachgewiesen, wobei als Vergleichssubstanzen die anerkannt gut wirksamen bekannten Handelsprodukte 5,5-Diphenylhydantoin [Vergleichssubstanz A] und 3-Methyl-5-äthyl-5-phenylhydantoin [Vergleichssubstanz B] gleicher Wirkungsrichtung verwendet wurden. Die Tierversuche wurden mit CFLP-Mäusen (LATI, Gödöllő, Ungarn) von gemischtem Geschlecht (Körpergewicht: 18 bis 22 g) mit Gruppen von je 10 Tieren durchgeführt. Die zu untersuchenden Verbindungen wurden in einer 5%-igen wäßrigen Lösung von

Polyoxyäthylensorbitanmonooleat [Tween 80®] suspendiert und diese Suspensionen wurden auf peroralem Wege mit einer Sonde den Tieren verabreicht. Die Wirkstoffe wurden in den zur Untersuchung der krampfhemmenden beziehungsweise antikonvulsiven Wirkung durchgeführten Versuchen in Dosen von 20 mg/kg Körpergewicht und zur Messung der neurotoxischen Wirkung in Dosen von 80 mg/kg Körpergewicht verabreicht. Die Wirkungen wurden 1 Stunde nach der Verabreichung durch die im folgenden beschriebenen Verfahrensweisen gemessen.

## I) Prüfung der krampfhemmenden beziehungsweise antikonvulsiven Wirkung

### a) Maximaler Elektroschock [MES]

Die Tiere wurden nach der Verfahrensweise von E. A. Swinyard und Mitarbeitern (J. Pharmacol. 106 [1952], 319) mit einer Hornhautelektrode beziehungsweise Corneal-Elektrode (20 mA, 0,2 Sekunde) geschockt. 100% der Blindversuchs- beziehungsweise Kontrolltiere reagierten auf diesen Reiz mit einem tonischen Streckkrampf (extensorischen Krampf) der hinteren Gliedmaßen. Das Unterbleiben dieser Reaktion wurde als unter der Wirkung der Behandlung eingetretener Schutz gewertet.

### b) Hemmwirkung gegen den Pentamethylentetrazolkrampf [Pentetrazolkrampf]

Es wurden 125 mg Pentamethylentetrazol/kg Körpergewicht nach der Verfahrensweise von Cr. N. Everett und R. K. Richards (J. Pharmacol. Exp. Therap. 81 [1944], 402) den Tieren subkutan verabreicht. 1 Stunde nach der Verabreichung des zu untersuchenden Wirkstoffes wurde die Wirkung beobachtet: Als Schutzwirkung wurde das Unterbleiben des klonischen Krampfes beziehungsweise des tonischen

Streckkrampfes der hinteren Gliedmaßen bewertet.

## II) Prüfung der neurotoxischen Wirkung

### Messung der Muskelinkoordination [RR] an Mäusen

Die Veränderung der koordinierten Muskelbewegung wurde nach der Verfahrensweise von W. J. Kinnard und C. F. Carr (Brit. J. Pharmacol. 121 [1957], 354) an einem gedrehten Stab (Durchmesser: 20 mm; Frequenz: 12 Umdrehungen/Minute) geprüft. Die normal trainierten Tiere konnten 120 Sekunden lang am gedrehten Stab bleiben. Es wurde 1 Stunde nach der Verabreichung der zu untersuchenden Wirkstoffe beobachtet, wie viele Tiere innerhalb 120 Sekunden vom gedrehten Stab gefallen sind, also bei wieviel Prozent der Tiere eine Muskelinkoordination aufgetreten ist.

## III) Akute Toxizität

Die Toxizität der zu untersuchenden Verbindungen wurde nach 1-maliger Verabreichung von verschiedenen Dosen durch 14 Tage lange Beobachtung der Tiere ermittelt. Die $LD_{50}$-Werte (die bei 50% der Tiere tödlichen Dosen) wurden auf Grund des Prozentsatzes der innerhalb 14 Tage verendeten Tiere berechnet.

Die erhaltenen Ergebnisse sind in der folgenden Tabelle 1 zusammengestellt.

Tabelle 1

| Verbindungen | | | | | | | Hemmwirkung in % in Bezug auf den | | | Muskelinkoordination in % | Peroraler $LD_{50}$-Wert in mg/kg |
|---|---|---|---|---|---|---|---|---|---|---|---|
| Beispiel Nr. | Symbole | | | | | | maximalen Elektroschock | Pentamethylentetrazolkrampf | | | |
| | $R_2$ | $R_3$ | $R_3'$ | $R_4$ | $R_6$ | $R_6'$ | | tonischen Streckkrampf | klonischen Krampf | | |
| 63 | H | 4-Hydroxyphenyl | H | H | H | H | 40 | 20 | - | 20 | 800 |
| 2 | Acetyl | Phenyl | H | H | H | H | 70 | 90 | - | 20 | 1 188 |
| 89 | Äthoxycarbonyl | Phenyl | H | H | H | H | - | 20 | - | - | 1 000 |
| 83 | Acetyl | Pentamethylen | | H | H | H | 40 | - | - | - | 1 000 |
| 84 | Acetyl | 4-Carboxyphenyl | H | H | H | H | 40 | - | - | - | 1 000 |
| 13 | Acetyl | 4-Fluorphenyl | H | H | H | H | 40 | 40 | - | - | 800 |

Fortsetzung der Tabelle 1

| Verbindungen | | | | | | | Hemmwirkung in % in Bezug auf den | | | Muskel-inkoordination in % | Peroraler $LD_{50}$-Wert in mg/kg |
|---|---|---|---|---|---|---|---|---|---|---|---|
| Beispiel Nr. | Symbole | | | | | | maximalen Elektroschock | Pentamethylentetrazolkrampf | | | |
| | $R_2$ | $R_3$ | $R_3'$ | $R_4$ | $R_6$ | $R_6'$ | | tonischen Streckkrampf | klonischen Krampf | | |
| 14 | Acetyl | 4-Bromphenyl | H | H | H | H | 40 | - | - | - | 800 |
| 15 | Acetyl | α-Naphthyl | H | H | H | H | 40 | - | - | - | 800 |
| 16 | Acetyl | Thien-2-yl | H | H | H | H | 20 | 40 | - | 20 | 800 |
| 8 | Acetyl | Phenyl | H | Acetyl | H | H | 60 | 40 | - | 20 | 1 000 |
| 61 | Acetyl | Phenyl | H | H | DL-Methyl | H | 20 | 50 | - | - | 1 000 |
| 57 | Acetyl | Phenyl | H | H | L-Methyl | H | 40 | 60 | - | 20 | 800 |
| 60 | Acetyl | Phenyl | H | H | DL-Benzyl | H | - | 20 | - | 40 | 1 000 |

Fortsetzung der Tabelle 1

| Verbindungen Beispiel Nr. | Symbole $R_2$ | $R_3$ | $R_3'$ | $R_4$ | $R_6$ | $R_6'$ | Hemmwirkung in % in Bezug auf den maximalen Elektroschock | Pentamethylentetrazolkrampf: tonischen Streckkrampf | Pentamethylentetrazolkrampf: klonischen Krampf | Muskelinkoordination in % | Peroraler $LD_{50}$-Wert in mg/kg |
|---|---|---|---|---|---|---|---|---|---|---|---|
| 59 | Acetyl | Phenyl | H | H | L-Benzyl | H | - | 20 | - | 20 | 1 000 |
| Vergleichssubstanz A | Diphenylhydantoin | | | | | | 90 | 80 | - | 70 | 279 |
| Vergleichssubstanz B | 3-Methyl-5-äthyl-5-phenylhydantoin | | | | | | 10 | 50 | - | 50 | 476 |

Aus den Daten der obigen Tabelle 1 geht hervor, daß die erfindungsgemäßen Tetrahydro-1,2,4-oxadiazin-5-onderivate der allgemeinen Formel I überlegene krampfhemmende beziehungsweise antikonvulsive Wirkungen haben, wobei ihre neurotoxische Wirkung unbedeutend ist (geringere Werte der Muskelinkoordination als bei den Vergleichssubstanzen) und mit ihnen tödliche Wirkungen nur bei sehr hohen Dosen auftreten (höhere $LD_{50}$-Werte als die der Vergleichssubstanzen), so daß sie bei höheren therapeutischen Indices eine wesentlich größere therapeutische Breite haben als die bekannten Verbindungen gleicher Wirkungsrichtung. Auf Grund dieser vorteilhaften Eigenschaften können die erfindungsgemäßen Tetrahydro-1,2,4-oxadiazin-5-onderivate der allgemeinen Formel I vorteilhafter als die auf diesem Gebiet in weitem Umfang verwendeten bekannten Hydantoinderivate zur Behandlung von epileptischen Erkrankungen eingesetzt werden.

Die erfindungsgemäßen Tetrahydro-1,2,4-oxadiazin-5-onderivate können in der Therapie in Form von üblichen Arzneimittelpräparaten verwendet werden. Solche Arzneimittelpräparate können das beziehungsweise die erfindungsgemäße(n) Tetrahydro-1,2,4-oxadiazin-5-onderivat(e) zusammen mit 1 oder mehr an sich bekannten zur enteralen oder parenteralen Verabreichung geeigneten festen oder flüssigen anorganischen oder organischen Trägerstoff(en) und/oder Hilfsstoff(en) enthalten. Sie können beispielsweise in Form von Tabletten, Injektionslösungen, verdünnten oder konzentrierten Suspensionen oder Emulsionen zubereitet sein.

Diese Arzneimittelpräparate können in einer Dosierungseinheit (wie Tablette oder Ampulle) zweckmäßig Mengen von 30 bis 100 mg des beziehungsweise der jeweiligen erfindungsgemäßen Tetrahydro-1,2,4-oxadiazin-5-onderivate[s] der allgemeinen Formel I als Wirkstoff(e) enthalten.

Die erfindungsgemäßen Arzneimittel können in der Humantherapie peroral oder parenteral, insbesondere intravenös, verabreicht werden. Die tägliche Dosis der Wirkstoffe Tetrahydro-1,2,4-oxadiazin-5-onderivate kann bei erwachsenen Menschen unter Berücksichtigung der Art der Krankheit, des Zustandes der Patienten und der gewünschten Wirkung im allgemeinen 200 bis 600 mg betragen, wobei diese Tagesdosen auf einmal oder während des Tages auf mehrere kleine Dosen verteilt verabreicht werden können.

Die Erfindung wird an Hand der folgenden Beispiele näher erläutert.

Die in den Beispielen angegebenen Schmelzpunkte wurden in einem Schmelzpunktmeßgerät nach Dr. Tottoli [Büchi] bestimmt. Die Dünnschichtchromatogramme wurden auf gegen UV-Licht sensibilisierten Silicagelplatten ("Kieselgel G® nach Stahl" [Merck]) hergestellt. Zur Entwicklung der Chromatogramme wurden die folgenden Lösungsmittelgemische verwendet:

| | Lösungsmittelgemisch | Volumverhältnis |
|---|---|---|
| /A/: | Benzol/Aceton | 1 : 1 |
| /B/: | Chloroform/Methanol | 3 : 1 |
| /C/: | Äthylacetat/Essigsäure/Wasser/Pyridin | 30 : 4 : 2 : 1 |
| /D/: | n-Hexan/Essigsäure/Chloroform | 1 : 4 : 8 |
| /E/: | Äthylacetat/Essigsäure/Wasser/Pyridin | 63 : 4 : 2 : 1 |
| /F/: | Chloroform/Methanol | 1 : 1 |

Die Entwicklung der Dünnsichtchromatogramme wurde in der Mehrzahl der Fälle nach einer der folgenden Verfahrensweisen oder Kombinationen derselben durchgeführt:

I. UV-Bestrahlung (Wellenlänge: 254 nm).

II. Behandlung mit Joddämpfen.

III. Chlorierung und nachfolgendes Besprühen mit Tolidin/Kaliumjodid.

Die Identifizierung der Struktur der hergestellten Verbindungen erfolgte auf Grund der Elementaranalysen, der Ultrarotspektren (IR-Spektren) und der magnetischen Kernresonanzspektren (NMR-Spektren) der Verbindungen. Die Ultrarotspektren wurden in einem Apparat vom Typ "Perkin-Elmer 257" und die magnetischen Kernresonanzspektren in einem magnetischen Kernresonanzspektrometer vom Typ "Varion EM-60" aufgenommen.

Bei der Aufarbeitung der Reaktionsgemische wurden die Lösungen der erhaltenen Produkte in einem Vakuumeindampfer vom Typ "Rotavapor R" [Büchi] bei 50°C nicht übersteigenden Temperaturen eingedampft.

Bei den Daten der Ultrarotspektren bedeutet Z den Benzyloxycarbonylrest.

Bei der Aufnahme der magnetischen Kernresonanzspektren wurden diese, sofern sie in mit Wasser nicht mischbaren Lösungsmitteln, zum Beispiel in Deuterochloroform, aufgenommen wurden, auch nach Ausschütteln mit schwerem Wasser aufgenommen, wobei dann die Signale der gegen Deuterium leicht austauschbaren Protonen aus den Spektren verschwunden sind (dieser Umstand wurde in den entsprechenden Beispielen durch einen Stern [*] angedeutet) und die Multiplizität der mit diesen verknüpften Protonen vereinfacht wurde (dieser

Umstand wurde im Text ebenfalls angedeutet; so ist zum Beispiel das Symbol "d → s" so zu verstehen, daß das ursprüngliche Dublett sich in ein Singulett umgewandelt hat).

Beispiel 1

2-Benzyloxycarbonyl-3-phenyl-tetrahydro-1,2,4-oxadiazin-5-on

nach der Variante a)
[Verfahrensweise A]

10,1 g /45 mMol/ α-(Benzyloxycarbonyl-aminooxy)-acetamid, 5,05 ml /650 mMol/ frisch destillierter Benzaldehyd und 1,0 g dl-Campher-10-sulfonsäure wurden in 200 ml Benzol in einem mit einem Marcusson-Aufsatz ausgerüsteten Kolben 8 bis 10 Stunden lang gekocht, wobei der Verbrauch der Ausgangsverbindung durch Dünnschichtchromatographie verfolgt wurde. Nach der Beendigung der Reaktion wurde das Reaktionsgemisch zur Trockne eingedampft und der Rückstand aus 80 ml Äthanol umkristallisiert. Es wurden auf diese Weise 10,6 g 2-Benzyloxycarbonyl-3-phenyl-tetrahydro-1,2,4-oxadiazin-5-on /76 % d. Th./ erhalten; F. 130 133°C; $R_f^{/A/}$ = 0,7.

Analyse für $C_{17}H_{16}N_2O_4$ /Mol.Gew.: 321,33/:

berechnet: C 65,38 %, H 5,16 %, N 8,97 %;
gefunden: C 65,11 %, H 5,43 %, N 8,55 %.

IR /KBr/ $cm^{-1}$: 3180 /-NH-/, 1740 /C=O, Z/, 1685 /C=O, Amid/, 1412, 824 /Ring/, 1583, 748, 698 /aromatisch/.

NMR /DMSO-$D_6$ + $CDCl_3$, TMS/ ppm: 4,48 AB q /-$CH_2$-$C_6H_5$/, 6,55 breites s />CH-NH-/, 7,40 s /10 H, aromatisch/, 8,90 breit /-NH-/.

Beispiel 2

2-Acetyl-3-phenyltetrahydro-1,2,4-oxadiazin-5-on

nach der Variante a)
[Verfahrensweise B]

Es wurde ein Gemisch von 1,32 g (0,01 Mol) N-(Acetyl)-aminooxyacetamid, 1,1 ml (1,06 g, 0,01 Mol) Benzaldehyd, 0,2 g Campher-10-sulfonsäure, 10 ml Toluol und 10 ml Butylacetat 1 Stunde lang unter Rückfluß zum Sieden erhitzt. Dann wurde noch 0,5 ml Benzaldehyd zugesetzt und das Kochen eine weitere Stunde fortgesetzt. Während des Abkühlens schied sich das Produkt in kristalliner Form aus dem Reaktionsgemisch aus. Das kristalline Produkt wurde abfiltriert und 2-mal mit je 10 ml Äther gewaschen. So wurden 1,12 g (55% der Theorie) 2-Acetyl-3-phenyltetrahydro-1,2,4-oxadiazin-5-on mit einem Schmelzpunkt von 167 bis 168°C und einem $R_f^{/B/}$-Wert von 0,7 erhalten.

Analyse für $C_{11}H_{12}N_2O_3$ /Mol.Gew.: 220,23/:

berechnet: C 59,99 %, H 5,49 %, N 12,72 %;
gefunden: C 60,12 %, H 5,92 %, N 12,73 %.

IR /KBr/ $cm^{-1}$: 3180 /-NH-/, 1690, 1668 /C=O, Amid/, 1412, 790 /Ring/, 740, 700 /aromatisch/.

NMR /DMSO-$d_6$ + $CDCl_3$, TMS/ ppm: 2,13 s /-$CH_3$/, 4,56 AB q /-$CH_2$/, 6,70 breit, s /-CH-/, 7,45 s /5H, aromatisch/, 9,2 breit /-NH-/.

Beispiel 3

2-Acetyl-3-phenyl-tetrahydro-1,2,4-oxadiazin-5-on

nach der Variante a)
[mit Herstellung aus der Ausgangsverbindung in situ]

Es wurde wie im nächsten Absatz beschrieben erhaltenes

rohes N-(Acetyl)-aminooxyacetamid in einem Gemisch von 500 ml Essigsäure und 61,5 ml Essigsäureanhydrid suspendiert, die Suspension wurde mit 61,5 g (0,67 Mol) Benzaldehyd und anschließend mit 25 ml konzentrierter Schwefelsäure versetzt, wobei die Zugabe der Schwefelsäure in kleinen Portionen in einem solchen Tempo erfolgte, daß die Temperatur des Gemisches während der Zugabe 30°C nicht überstieg. Das Gemisch wurde 1 Stunde bei Raumtemperatur gerührt beziehungsweise geschüttelt, dann wurden 140 g kristallines Natriumacetat zugesetzt und nach noch 10 Minuten langem Rühren beziehungsweise Schütteln wurde das Lösungsmittel unter vermindertem Druck abgedampft. Der Rückstand wurde in 1 l Äthylacetat gelöst, die Lösung wurde mit 250 ml Wasser ausgeschüttelt, über wasserfreiem Natriumsulfat getrocknet und zur Trockne eingedampft. Der Rückstand wurde aus 100 ml Äthanol umkristallisiert. So wurden 28 g (25,4% der auf Hydroxylamin bezogenen theoretischen Ausbeute) 2-Acetyl-3--phenyltetrahydro-1,2,4-oxadiazin-5-on mit einem Schmelzpunkt von 165 bis 166°C und einem $R_f^{/B/}$-Wert von 0,7 erhalten. Die Ultrarotspektren und magnetischen Kernresonanzspektren des Produktes hatten die gleichen Werte wie die im Beispiel 2 angegebenen.

Es wurden 34,75 g (0,5 Mol) Hydroxylaminhydrochlorid in 500 ml Methanol gelöst und dann wurde die Lösung mit 55 g (0,52 Mol) wasserfreiem Natriumcarbonat und 75,5 ml (0,78 Mol) Äthylacetat versetzt. Das Gemisch wurde 5 Stunden unter Rückfluß zum Sieden erhitzt und dann über Nacht stehengelassen. Zum die entstandene Acethydroxamsäure enthaltenden Reaktionsgemisch wurden 46,7 g (0,5 Mol) Chloracetamid zugegeben und das Gemisch wurde 5 Stunden unter Rühren beziehungsweise Schütteln und unter Rückfluß zum Sieden erhitzt, dann wurde es abgekühlt und das Lösungsmittel wurde unter vermindertem Druck abgedampft. Als Rückstand wurde auch anorganische Salze enthaltendes rohes N-(Acetyl)-aminooxyacetamid erhalten. Dieses rohe Produkt konnte ohne Reinigung

in der nächsten Reaktionsstufe [Umsetzung mit Benzaldehyd nach der Variante a) des erfindungsgemäßen Verfahrens] verwendet werden, gegebenenfalls konnte es aber für analytische Zwecke durch Entsalzen mit Hilfe eines Ionenaustauscherharzes gereinigt werden. Das reine Produkt N-(Acetyl)-aminooxyacetamid hatte einen Schmelzpunkt von 88 bis 90°C.

Beispiel 4

2-Benzyloxycarbonyl-3-n-propyl-tetrahydro-1,2,4-oxadiazin-5-on

nach der Variante a)
[Verfahrensweise C]

Das Gemisch von 45 g /0,2 Mol/ α-(Benzyloxycarbonyl-aminooxy)-acetamid, 23 ml /20,2 g, 0,28 Mol/ Butyraldehyd, 200 ml Essigsäure, 26 ml Essigsäureanhydrid und 10 ml konz. Schwefelsäure wurde bei Raumtemperatur 24 Stunden stehengelassen, dann wurden weitere 5 ml Butyraldehyd zugesetzt und das Gemisch weitere 2 Tage bei Raumtemperatur stehengelassen. Anschließend wurde das Reaktionsgemisch mit 56 g Natriumacetat-trihydrat versetzt, mit Aktivkohle entfärbt und filtriert. Das Filtrat wurde eingedampft, der Rückstand mit dem Gemisch von 100 ml Wasser und 100 ml Diisopropyläther vermischt, das gefällte feste Produkt durch Filtrieren abgetrennt und abwechselnd mit Wasser und mit Diisopropyläther mehrmals gewaschen. Es wurden auf diese Weise 32 g 2-Benzyloxycarbonyl-3-n-propyl-tetrahydro-1,2,4-oxadiazin-5-on /57 % d. Th./ erhalten, F. 105 - 106°C.

Analyse für $C_{14}H_{18}N_2O_4$ /Mol.Gew.: 278,31/:
berechnet: C 60,42 %, H 6,52 %, N 10,07 %;
gefunden: C 60,69 %, H 6,60 %, N 9,83 %.
IR /KBr/ $cm^{-1}$: 3180 /-NH-/, 1730 /$\geq$C=O, Z/, 1680 /$\geq$C=O, Amid/, 1422, 790 /Ring/, 755, 695 /aromatisch/.
$^1$H-NMR /DMSO-$d_6$ + $CDCl_3$, TMS/ ppm: 0,8 - 2,2 m /-$C_3H_7$/, 4,30 AB q /-$CH_2$-C=O/, 5,27 s /-$CH_2$-$C_6H_5$/, 7,44 s /5H, aromatisch/, 8,80 breit /-NH-/.

Beispiel 5

3-Phenyl-tetrahydro-1,2,4-oxadiazin-5-on

nach der Variante b)
[Verfahrensweise D]

6,8 g /21,8 mMol/ 2-Benzyloxycarbonyl-3-phenyl-tetrahydro-1,2,4-oxadiazin-5-on wurden im Gemisch von 100 ml Methanol und 125 ml Äthylacetat gelöst, die Lösung wurde mit 1,0 g 5 %-igem Palladium-Aktivkohle-Katalysator versetzt und Wasserstoffgas wurde durch das Gemisch geleitet. Das Fortschreiten der Reaktion wurde durch Dünnschichtchromatographie auf Grund des Verbrauchens der Ausgangsverbindung verfolgt. Nach der Beendigung der Reaktion wurde der Katalysator abfiltriert und das Filtrat zur Trockne eingedampft. Der Rückstand wurde aus dem Gemisch von 10 ml Äthylacetat und 30 ml n-Hexan umkristallisiert; es wurden auf diese Weise 3,0 g 3-Phenyl-tetrahydro-1,2,4-oxadiazin-5-on /77 % d. Th./ erhalten; F. 118 - 120°C; $R_f^{/A/}$ = 0,35.

Analyse für $C_9H_{10}N_2O_2$ /Mol.Gew.: 178,19/:

berechnet: C 60,66 %, H 5,66 %, N 15,72 %.
gefunden: C 60,33 %, H 5,57 %, N 15,94 %.
IR /KBr/ $cm^{-1}$: 3160 /-NH-/, 1670 />C=O/, 1420, 806 /Ring/, 700 /aromatisch/.
NMR /DMSO-$d_6$ + $CDCl_3$, TMS/ ppm: 4,22 s /-$CH_2$-/, 5,53 d → s, J = 6,5 Hz />CH-/, 7,02 d*, J = 6,5 Hz /-NH-/, 7,47 s /5H, aromatisch/, 8,80 breit* /-NH-/.

Beispiel 6

2-Acetyl-3-phenyl-tetrahydro-1,2,4-oxadiazin-5-on

nach der Variante c)
[Verfahrensweise E]

38,5 g /216 mMol/ 3-Phenyl-tetrahydro-1,2,4-oxadiazin-5-on wurden in 190 ml Essigsäureanhydrid eine Stunde gerührt. Die anfängliche Suspension ging rasch in eine Lösung über und dann begann die Ausscheidung des Produkts. Nach der Beendigung der Reaktion wurde das Lösungsmittel unter vermindertem Druck verdampft und der Rückstand aus 150 ml Äthanol umkristallisiert. Es wurden auf diese Weise 36,3 g 2-Acetyl-3-phenyl-tetrahydro-1,2,4-oxadiazin-5-on /81 % d. Th./ erhalten; F. 167 - 168°C; $R_f^{/B/}$ = 0,7.

Analyse für $C_{11}H_{12}N_2O_3$ /Mol.Gew.: 220,23/:

berechnet: C 59,99 %, H 5,49 %, N 12,72 %;
gefunden: C 60,12 %, H 5,92 %, N 12,73 %.
IR /KBr/ $cm^{-1}$: 3180 /-NH-/, 1690, 1668 />C=O, Amid/, 1412, 790 /Ring/, 740, 700 /aromatisch/.
NMR /DMSO-$d_6$ + $CDCl_3$, TMS/ ppm: 2,13 s /-$CH_3$/, 4,56 AB q /-$CH_2$-/, 6,70 breites s />CH-/, 7,45 s /5H, aromatisch/, 9,2 breit* /-NH-/.

Beispiel 7

2-Acetyl-3-phenyl-tetrahydro-1,2,4-oxadiazin-5-on

nach der Variante b)
[Verfahrensweise F]

6,28 g /20 mMol/ 2-Benzyloxycarbonyl-3-phenyl-tetrahydro-1,2,4-oxadiazin-5-on wurden in 50 ml Essigsäureanhydrid gelöst, die Lösung wurde mit 0,5 g 5 %-igem Palladium-Aktivkohle-Katalysator versetzt und Wasserstoffgas wurde durch die Lösung geleitet. Das Fortschreiten der Reaktion wurde durch Dünnschichtchromatographie verfolgt; nach der Beendigung der Reaktion wurde der Katalysator abfiltriert, das Filtrat zur Trockne eingedampft und der Rückstand aus 15 ml Äthanol umkristallisiert. Es wurden auf diese Weise 3,55 g 2-Acetyl-3-phenyl-tetrahydro-1,2,4-oxadiazin-5-on /80 % d. Th./ erhalten; die physikalischen Konstanten des Produkts sind mit denen des nach Beispiel 6 erhaltenen Produkts identisch.

Beispiel 8

2,4-Diacetyl-3-phenyltetrahydro-1,2,4-oxadiazin-5-on

nach der Variante e)

Es wurden 1,1 g (5 Millimol) 2-Acetyl-3-phenyltetrahydro-1,2,4-oxadiazin-5-on in 10 ml wasserfreiem Tetrahydrofuran gelöst, die Lösung wurde mit 1,5 ml Triäthylamin versetzt, dann auf 0°C abgekühlt und bei dieser Temperatur mit 0,8 ml (11 Millimol) Acetylchlorid versetzt. Das Gemisch wurde 20 Stunden lang unter Rückfluß zum Sieden erhitzt und nach dem Abkühlen wurde das Triäthylaminsalz abfiltriert und das Filtrat zur Trockne eingedampft. Der Rückstand wurde auf eine Silicagelsäule gebracht und durch Eluieren mit einem Gemisch von Benzol und Aceton im Volumverhältnis von 1 : 1 gereinigt. Die das gewünschte Produkt enthaltende

Fraktion wurde eingedampft und der Rückstand wurde aus einem Gemisch von 2 ml Äthylacetat und 6 ml n-Hexan umkristallisiert. So wurde 0,3 g (23% der Theorie) 2,4-Diacetyl-3--phenyltetrahydro-1,2,4-oxadiazin-5-on mit einem Schmelzpunkt von 104 bis 106°C und einem $R_f^{/A/}$-Wert von 0,8 erhalten.

Analyse für $C_{13}H_{14}N_2O_4$ /Mol.Gew.: 262,27/:

| | | | |
|---|---|---|---|
| berechnet: | C 59,54 %, | H 5,38 %, | N 10,68 %; |
| gefunden: | C 59,30 %, | H 5,12 %, | N 10,84 %. |

IR /KBr/ $cm^{-1}$: 1710, 1680, 1648 /$\geq$C=O/, 1585, 755, 700 /aromatisch/.

NMR /DMSO-$d_6$ + $CDCl_3$, TMS/ ppm: 2,13 s /-$CH_3$/, 2,56 s /-$CH_3$/, 4,8 AB q /-$CH_2$-/, 7,36 s /5H, aromatisch/, 7,5 s /$\geq$CH-/.

Beispiel 9

2-Phenoxycarbonyl-3-n-propyl-tetrahydro-1,2,4--oxadiazin-5-on

nach der Variante c)
[Verfahrensweise G]

11,25 ml /13,9 g, 89 mMol/ Phenoxycarbonylchlorid wurden in 75 ml wasserfreiem Tetrahydrofuran gelöst, die Lösung wurde auf 0°C abgekühlt und bei dieser Temperatur mit der Lösung von 10,8 g /75 mMol/ 3-n-Propyl-tetrahydro-1,2,4--oxadiazin-5-on im Gemisch von 150 ml wasserfreiem Tetrahydrofuran und 11,4 ml wasserfreiem Triäthylamin tropfenweise versetzt. Nach der Beendigung der Zugabe wurde das Gemisch auf Raumtemperatur gebracht und 3 Stunden gerührt. Das ausgeschiedene Triäthylamin-hydrochlorid wurde abfiltriert und das Filtrat unter vermindertem Druck eingedampft. Der Rückstand wurde mit einem Gemisch von Wasser und Äther im Volumverhältnis von 1 : 1 verrieben und abfiltriert. Es wurden

auf diese Weise 14,7 g 2-Phenoxycarbonyl-3-n-propyl-tetrahydro-1,2,4-oxadiazin-5-on /80% d. Th./ erhalten, F. 106 - 107°C.

Analyse für $C_{13}H_{16}N_2O_4$ /Mol.Gew.: 264,28/:

berechnet: C 59,08 %, H 6,10 %, N 10,60 %;
gefunden: C 59,31 %, H 5,70 %, N 10,70 %.

IR /KBr/ $cm^{-1}$: 3180 /-NH-/, 1735 />C=O, Z/, 1680 />C=O, Amid/, 1210 />C-O-C</, 1592, 690, 742 /aromatisch/.

NMR /DMSO-$d_6$ + $CDCl_3$, TMS/ ppm: 0,95 /-$CH_3$/, 1,44 m /-$\underline{CH_2}$-$CH_3$/, 2,10 m />CH-$\underline{CH_2}$-/, 4,5 AB q /$\underline{CH_2}$-CO-/, 5,3 m → t />CH-/, 7,5 m /5H, aromatisch/, 9,0 d* /-NH-/.

Beispiel 10

2-Carbamoyl-3-n-propyl-tetrahydro-1,2,4-oxadiazin-5-on

nach der Variante f)
[Verfahrensweise H]

7,94 g /30 mMol/ 2-Phenoxycarbonyl-3-n-propyl-tetrahydro-1,2,4-oxadiazin-5-on wurden im Gemisch von 75 ml Chloroform und 75 ml konzentrierter Ammoniumhydroxydlösung bei Raumtemperatur lebhaft gerührt, bis in den entnommenen Proben der organischen Phase durch Dünnschichtchromatographie keine ~~nicht umgesetzte~~ Ausgangsverbindung mehr nachgewiesen werden konnte /was etwa 1-2 Tage in Anspruch genommen hat/. Nach der Beendigung der Reaktion wurde das ausgeschiedene Produkt durch Filtrieren abgetrennt, mit Wasser und mit Äther gewaschen und getrocknet.

Aus dem auf obige Weise erhaltenen Filtrat wurde die organische Phase abgetrennt, mit wasserfreiem Natriumsulfat getrocknet und eingedampft. Der Rückstand wurde mit Äther

verrieben und abfiltriert. Die auf diese Weise erhaltene weitere Menge des Produkts wurde mit der Hauptmenge vereinigt, es wurden auf diese Weise insgesamt 4,6 g 2-Carbamoyl-3-n-propyl-tetrahydro-1,2,4-oxadiazin-5-on /82 % d. Th./ erhalten; F. 189°C.

Beispiel 11

2-/n-Butyl-carbamoyl/-3-n-propyl-tetrahydro-1,2,4--oxadiazin-5-on

nach der Variante g)

3,0 g /20,8 mMol/ 3-n-Propyl-tetrahydro-1,2,4-oxadiazin-5-on wurden in 40 ml wasserfreiem Tetrahydrofuran gelöst, die Lösung wurde unter 5°C abgekühlt und bei dieser Temperatur mit der Lösung von 2,5 ml /2,2 g, 22,5 mMol/ n-Butyl-isocyanat in 20 ml wasserfreiem Tetrahydrofuran tropfenweise versetzt. Das Gemisch wurde bei Raumtemperatur 4 Tage lang gerührt. Als auf dem Dünnschichtchromatogramm der Fleck der Ausgangsverbindung nicht mehr sichtbar war, wurde das Reaktionsgemisch im Vakuum zur Trockne eingedampft, der Rückstand mit n-Hexan verrieben, abfiltriert und aus einem Gemisch von Äthylacetat und n-Hexan im Volumverhältnis von 1 : 3 umkristallisiert. Es wurden auf diese Weise 4,23 g 2-/n-Butyl-carbamoyl/-3-n-propyl-tetrahydro-1,2,4--oxadiazin-5-on /87% d. Th./ erhalten; F. 105 - 106°C.

Analyse für $C_{11}H_{21}N_3O_3$ /Mol.Gew.: 243,31/:

berechnet: C 54,30 %, H 8,70 %, N 17,27 %;

gefunden: C 54,68 %, H 8,77 %, N 17,54 %.

IR /KBr/ $cm^{-1}$: 3320, 3180 /-NH-/, 1690 /$\geq$C=O, Carbamoyl/, 1660 /$\geq$C=O, Amid/, 1421, 820 /Ring/.

$^1H$-NMR /DMSO-$d_6$ + $CDCl_3$, TMS/ ppm: 0,7-1,9 m /14H, aliphatisch/, 3,15 q → t /-NH-$\underline{CH_2}$-/, 4,21 q /-O-$\underline{CH_2}$-/, 5,30 m → t />CH-/, 7,13 d*, J = 6 Hz /-NH-/, 8,70 d, J = 3 Hz /-NH-/.

Beispiel 12

2-Formyl-3-phenyl-tetrahydro-1,2,4-oxadiazin-5-on

nach der Variante d)

5,4 g /30 mMol/ 3-Phenyl-tetrahydro-1,2,4-oxadiazin--5-on und 10,5 g /51 mMol/ Dicyclohexyl-carbodiimid wurden in 50 ml wasserfreiem Tetrahydrofuran gelöst, die Lösung wurde auf nicht mehr als -5°C abgekühlt und bei dieser Temperatur mit 2,3 ml /2,35 g, 51 mMol/ Ameisensäure tropfenweise versetzt. Das Gemisch wurde bei -5°C 30 Minuten gerührt, dann wurde der ausgeschiedene Dicyclohexyl-harnstoff abfiltriert. Das Filtrat wurde im Vakuum zur Trockne eingedampft, der ölige Rückstand in heißem Wasser gelöst, die kleine Menge des ungelöst gebliebenen Stoffes wurde durch Filtrieren abgetrennt und das Filtrat wieder im Vakuum zur Trockne eingedampft. Der Rückstand wurde aus Äthylacetat umkristallisiert; es wurden auf diese Weise 1,52 g 2-Formyl-3-phenyl-tetrahydro-1,2,4-oxadiazin-5-on /24 % d. Th./ erhalten; F. 115 - 116°C; $R_f^{/D/}$ = 0,4.

Analyse für $C_{10}H_{10}N_2O_3$ /Mol. Gew.: 206,20/:

berechnet: C 58,25 %, H 4,89 %, N 13,59 %;
gefunden: C 58,30 %, H 4,69 %, N 13,59 %.

IR /KBr/ $cm^{-1}$: 3180 /-NH-/, 3050, 1700 /-CHO/, 1670 />C=O, Amid/, 1585, 755, 702 /aromatisch/.

$^1H$-NMR /DMSO-$d_6$ + $CDCl_3$, TMS/ ppm: 4,5 AB q /-$CH_2$-C=O/, 6,5 breit, s />CH-/, 7,3 breit /5H, aromatisch/, 8,5 s /-CHO/, 9,2 breit* /-NH-/.

Nach den in den obigen Beispielen beschriebenen Verfahrensweisen A bis H wurden auch die in der folgenden Tabelle 2 aufgezählten weiteren erfindungsgemäßen Tetrahydro-1,2,4-oxadiazin-5-onderivate der allgemeinen Formel I (Beispiele 13 bis 99) hergestellt. In dieser Tabelle 2 sind die einzelnen Verbindungen durch die Angabe der Symbole $R_2$ , $R_3$ , $R_3'$ , $R_4$ , $R_6$ und $R_6'$ definiert. In dieser Tabelle 2 sind neben den in den einzelnen Fällen verwendeten Herstellungsverfahrensweisen auch die Schmelzpunkte und $R_f$-Werte der einzelnen Verbindungen, die Ausbeuten an ihnen sowie die beim Umkristallisieren der Produkte verwendeten Lösungsmittel, die Reaktionsmedien und die bei der Herstellung der Verbindungen gegebenenfalls verwendeten Katalysatoren beziehungsweise Basen (soweit diese von denen, welche im die betreffende Verfahrensweise beschreibenden Beispiel verwendet wurden, verschieden sind) angegeben.

Tabelle 2

| Verbindung: Beispiel Nr. | Symbole: $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Katalysator beziehungsweise Base |
|---|---|---|---|---|---|---|---|---|---|---|---|---|---|---|
| 13 | Acetyl | 4-Fluorphenyl | H | H | H | H | 162 bis 163 | 0,4 /A/ | A | 5,8 | Äthanol | - | - | - |
| 14 | Acetyl | 4-Bromphenyl | H | H | H | H | | 0,4 /A/ | A | 20 | Äthylacetat | - | - | - |
| 15 | Acetyl | α-Naphthyl | H | H | H | H | 218 bis 220 | 0,8 /B/ | A | 28 | Äthanol | - | - | - |
| 16 | Acetyl | Thien-2-yl | H | H | H | H | 176 bis 177 | 0,8 /B/ | C | 20 | Äthylacetat | - | Essigsäure und Essigsäureanhydrid im Volumverhältnis von 1 : 1 | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 17 | Acetyl | 4-Nitrophenyl | H | H | H | H | 200 | 0,7 /B/ | A | 10 | Methanol | - | Benzol und Butylacetat im Volumverhältnis von 1 : 1 | - |
| 18 | Acetyl | 2-Carboxyphenyl | H | H | H | H | 188 bis 189 | 0,7 /B/ | A | 17 | Gemisch von Dioxan und Äthylacetat im Volumverhältnis von 1 : 1 | - | Benzol und Butylacetat im Volumverhältnis von 1 : 1 | - |
| 19 | Benzoyl | Phenyl | H | H | H | H | 153 bis 154 | 0,6 /A/ | A | 10 | - | - | | - |

Fortsetzung der Tabelle 2

| Verbindung | | | | | | | | | | | | | Anmerkung | |
|---|---|---|---|---|---|---|---|---|---|---|---|---|---|---|
| Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Reaktionsmedium abweichend | Katalysator beziehungsweise Base |
| 20 | Benzyloxycarbonyl | 2-Chlorphenyl | H | H | H | H | 135 bis 136 | 0,6 /A/ | B | 44 | Gemisch von Äthylacetat und n-Hexan im Volumverhältnis von 1 : 3 | - | Toluol | konzentrierte Schwefelsäure a) |
| 21 | Benzyloxycarbonyl | 4-Chlorphenyl | H | H | H | H | 147 bis 148 | 0,6 /A/ | B | 46 | Gemisch von Äthylacetat und n-Hexan im Volumverhältnis von 1 : 3 | - | Toluol | konzentrierte Schwefelsäure a) |

Fortsetzung der Tabelle 2

| Verbindung | | | | | | | Schmelzpunkt in °C | $R_f$-Wert | Verfahrensweise | Ausbeute in % der Theorie | Umkristallisationslösungsmittel | $\alpha_D^{20}$-Wert | Anmerkung | |
|---|---|---|---|---|---|---|---|---|---|---|---|---|---|---|
| Beispiel Nr. | $R_2$ | Symbole $R_3$ | $R_3'$ | $R_4$ | $R_6$ | $R_6'$ | | | | | | | Reaktionsmedium abweichend | Katalysator beziehungsweise Base |
| 22 | Benzyl-oxy-carbonyl | 2-Brom-phenyl | H | H | H | H | 140 bis 141 | 0,6 /A/ | B | 30 | - | - | Toluol | konzentrierte Schwefelsäure a) |
| 23 | Benzyl-oxy-carbonyl | 3-Brom-phenyl | H | H | H | H | 122 bis 123 | 0,4 /A/ | B | 47 | - | - | Toluol | konzentrierte Schwefelsäure a) |
| 24 | Benzyl-oxy-carbonyl | 4-Brom-phenyl | H | H | H | H | 158 bis 160 | 0,5 /A/ | B | 53 | Gemisch von Äthylacetat und n-Hexan im Volumverhältnis von 1 : 3 | - | Toluol | konzentrierte Schwefelsäure a) |

Fortsetzung der Tabelle 2

| Verbindung | | | | | | | Schmelzpunkt in °C | $R_f$-Wert | Verfahrensweise | Ausbeute in % der Theorie | Umkristallisationslösungsmittel | $\alpha_D^{20}$-Wert | Anmerkung | |
|---|---|---|---|---|---|---|---|---|---|---|---|---|---|---|
| Beispiel Nr. | Symbole | | | | | | | | | | | | Reaktionsmedium abweichend | Katalysator beziehungsweise Base |
| | $R_2$ | $R_3$ | $R_3'$ | $R_4$ | $R_6$ | $R_6'$ | | | | | | | | |
| 25 | Benzyloxycarbonyl | 4-Hydroxyphenyl | H | H | H | H | 193 bis 194 | 0,3 /A/ | A | 15 | Gemisch von Äthylacetat und n-Hexan im Volumverhältnis von 1 : 3 | – | – | – |
| 26 | Benzyloxycarbonyl | 4-Carboxyphenyl | H | H | H | H | 205 bis 210 | 0,8 /C/ | A | 61 | – | – | – | – |
| 27 | Benzyloxycarbonyl | 2-Carboxyphenyl | H | H | H | H | 166 bis 168 | 0,6 /B/ | A | 64 | – | – | – | – |

Fortsetzung der Tabelle 2

| Verbindung Bei-spiel Nr. | Symbole $R_2$ | $R_3$ | $R_3'$ | $R_4$ | $R_6$ | $R_6'$ | Schmelz-punkt in °C | $R_f$-Wert | Ver-fah-rens-weise | Aus-beute in % der Theorie | Umkristalli-sations-lösungs-mittel | $\alpha_D^{20}$-Wert | Anmerkung Reaktions-medium abweichend | Katalysator beziehungsweise Base |
|---|---|---|---|---|---|---|---|---|---|---|---|---|---|---|
| 28 | Benzyl-oxy-carbonyl | 4-Nitro-phenyl | H | H | H | H | 144 bis 145 | 0,3 /A/ | B | 46 | Äthanol | - | Toluol | - |
| 29 | Benzyl-oxy-carbonyl | 4-Dimethyl-amino-phenyl | H | H | H | H | 124 bis 127 | 0,7 /A/ | B | 20 | - | - | Toluol | - |
| 30 | Benzyl-oxy-carbonyl | 2,5-Di-methoxy-phenyl | H | H | H | H | 98 bis 100 | 0,5 /A/ | B | 42 | - | - | Toluol | konzentrierte Schwefel-säure a) |
| 31 | Benzyl-oxy-carbonyl | 2,4-Di-nitro-phenyl | H | H | H | H | 186 bis 187 | 0,8 /A/ | B | 17 | - | - | Toluol | konzentrierte Schwefel-säure a) |

Fortsetzung der Tabelle 2

| Verbindung: Beispiel Nr. | Symbole: $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Katalysator beziehungsweise Base |
|---|---|---|---|---|---|---|---|---|---|---|---|---|---|---|
| 32 | Benzyl-oxy-carbonyl | 3,4,5-Tri-methoxy-phenyl | H | H | H | H | 152 bis 153 | 0,7 /A/ | A | 88 | - | - | - | - |
| 33 | Benzyl-oxy-carbonyl | Phenyl | H | H | Äthyl | H | 127 bis 128 | 0,5 /A/ | A | 81 | Äthanol | - | - | - |
| 34 | Benzyl-oxy-carbonyl | 4-Chlor-phe-nyl | H | H | Äthyl | H | 118 bis 121 | 0,5 /A/ | B | 65 | Gemisch von Äthylacetat und n-Hexan im Volumverhältnis von 1 : 3 | - | Toluol | konzentrierte Schwefelsäure a) |

Fortsetzung der Tabelle 2

| Verbindung: Beispiel Nr. | Symbole: $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Katalysator beziehungsweise Base |
|---|---|---|---|---|---|---|---|---|---|---|---|---|---|---|
| 35 | Benzyl-oxy-carbonyl | 4-Brom-phe-nyl | H | H | Äthyl | H | 140 bis 143 | 0,5 /A/ | B | 68 | - | - | Toluol | konzentrierte Schwefel-säure a) |
| 36 | Benzyl-oxy-carbonyl | 3-Brom-phe-nyl | H | H | Äthyl | H | 121 bis 124 | 0,6 /A/ | B | 50 | - | - | Toluol | konzentrierte Schwefel-säure a) |
| 37 | Benzyl-oxy-carbonyl | 2-Brom-phe-nyl | H | H | Äthyl | H | 136 bis 138 | 0,6 /A/ | B | 49 | - | - | Toluol | konzentrierte Schwefel-säure a) |
| 38 | Benzyl-oxy-carbonyl | 2,5 Di-methoxy-phenyl | H | H | Äthyl | H | 141 bis 146 | 0,5 /A/ | B | 45 | - | - | Toluol | konzentrierte Schwefel-säure a) |

Fortsetzung der Tabelle 2

| Verbindung | | | | | | | Schmelzpunkt in °C | $R_f$-Wert | Verfahrensweise | Ausbeute in % der Theorie | Umkristallisationslösungsmittel | $\alpha_D^{20}$-Wert | Anmerkung | |
|---|---|---|---|---|---|---|---|---|---|---|---|---|---|---|
| Beispiel Nr. | $R_2$ | Symbole $R_3$ | $R_3'$ | $R_4$ | $R_6$ | $R_6'$ | | | | | | | Reaktionsmedium abweichend | Katalysator beziehungsweise Base |
| 39 | Benzyloxycarbonyl | Phenyl | H | H | Phenyl | H | 179 bis 180 | 0,8 /A/ | A | 65 | Äthanol | - | - | - |
| 40 | Benzyloxycarbonyl | 2,5-Dimethoxyphenyl | H | H | Phenyl | H | 160 bis 163 | 0,7 /A/ | A | 70 | Äthanol | - | - | - |
| 41 | Benzyloxycarbonyl | Phenyl | H | H | Phenyl | Phenyl | 162 bis 164 | 0,8 /A/ | B | 19 | - | - | Toluol | - |
| 42 | Benzyloxycarbonyl | Pentamethylen | | H | H | H | 158 bis 160 | 0,6 /A/ | A | 33 | Äthanol | - | - | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 43 | Benzyl-oxy-carbonyl | 5-Nitro-fur-2-yl | H | H | H | H | 152 bis 153 | 0,7 /A/ | C | 19 | Äthanol | - | - | - |
| 44 | Benzyl-oxy-carbonyl | Phenyl | H | H | DL-Methyl | H | 168 bis 170 | 0,7 /A/ | A | 68 | Äthanol | - | - | - |
| 45 | Benzyl-oxy-carbonyl | Phenyl | H | H | DL-Benzyl | H | 155 | 0,7 /A/ | A | 72 | Äthanol | - | - | - |
| 46 | Acetyl | 4-Brom-phenyl | H | H | D-Methyl | H | 160 bis 172 | 0,4 /A/ | B | 26 | - | +14,4° | Toluol | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Anmerkung: Katalysator beziehungsweise Base |
|---|---|---|---|---|---|---|---|---|---|---|---|---|---|---|
| 47 | Benzoyl | Phenyl | H | H | D-Methyl | H | 151 bis 153 | 0,7 /A/ | B | 76 | - | +134,0° | Toluol | - |
| 48 | Benzoyl | 4-Fluorphenyl | H | H | D-Methyl | H | 105 bis 108 | 0,6 /A/ | B | 55 | - | +175,0° | Toluol | - |
| 49 | Benzyloxycarbonyl | 4-Chlorphenyl | H | H | D-Methyl | H | 155 bis 159 | 0,5 /A/ | B | 25 | - | +5,8° | Toluol | konzentrierte Schwefelsäure a) |
| 50 | Benzyloxycarbonyl | 2-Bromphenyl | H | H | D-Methyl | H | 122 bis 124 | 0,5 /A/ | B | 23 | - | +85,8° | Toluol | konzentrierte Schwefelsäure a) |

Fortsetzung der Tabelle 2

| Verbindung Bei-spiel Nr. | Symbole $R_2$ | $R_3$ | $R_3'$ | $R_4$ | $R_6$ | $R_6'$ | Schmelz-punkt in °C | $R_f$-Wert | Ver-fah-rens-weise | Aus-beute in % der Theorie | Umkristalli-sations-lösungs-mittel | $\alpha_D^{20}$-Wert | Anmerkung Reaktions-medium abweichend | Katalysator beziehungsweise Base |
|---|---|---|---|---|---|---|---|---|---|---|---|---|---|---|
| 51 | Benzyl-oxy-carbonyl | 4-Nitro-phenyl | H | H | D--Me-thyl | H | 145 bis 147 | 0,5 /A/ | B | 17 | - | +2,3° | Toluol | konzentrierte Schwefel-säure a) |
| 52 | Benzyl-oxy-carbonyl | 4-Nitro-phenyl | H | H | D--Ben-zyl | H | 145 bis 147 | 0,5 /A/ | B | 37 | - | +144,0° | Toluol | konzentrierte Schwefel-säure a) |
| 53 | Benzyl-oxy-carbonyl | 2,5-Di-methoxy-phenyl | H | H | D--Me-thyl | H | 165 bis 174 | 0,5 /A/ | B | 59 | - | +5,8° | Toluol | konzentrierte Schwefel-säure a) |
| 54 | Benzyl-oxy-carbonyl | 2,5-Di-methoxy-phenyl | H | H | D--Ben-zyl | H | 126 bis 128 | 0,6 /A/ | B | 33 | - | +42,9° | Toluol | konzentrierte Schwefel-säure a) |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 55 | Benzyloxycarbonyl | 5-Nitrofur-2-yl | H | H | D-Methyl | H | - | 0,5 /A/ | B | 25 | - | +75,4° | Toluol | konzentrierte Schwefelsäure a) |
| 56 | Acetyl | Phenyl | H | H | D-Methyl | H | 194 bis 196 | 0,4 /D/ | F | 63 | - | +235° | - | - |
| 57 | Acetyl | Phenyl | H | H | L-Methyl | H | 193 bis 194 | 0,4 /D/ | F | 37 | Äthylacetat | -231° | - | - |
| 58 | Acetyl | Phenyl | H | H | D-Benzyl | H | 141 bis 143 | 0,7 /A/ | F | 42 | Methanol | +257° | - | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 59 | Acetyl | Phenyl | H | H | L-Benzyl | H | 146 bis 147 | 0,7 /A/ | F | 51 | Äthanol | -265° | - | - |
| 60 | Acetyl | Phenyl | H | H | DL-Benzyl | H | 173 bis 174 | 0,7 /A/ | F | 80 | Äthanol | - | Essigsäureanhydrid und Dimethylformamid im Volumverhältnis von 1 : 1 | - |
| 61 | Acetyl | Phenyl | H | H | DL-Methyl | H | 165 | 0,6 /A/ | F | 89 | Äthanol | - | Essigsäureanhydrid und Dimethylformamid im Volumverhältnis von 1 : 1 | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 62 | H | 2,5 Dimethoxyphenyl | H | H | H | H | 149 bis 150 | 0,45 /A/ | D | 91 | Äthanol | - | Dioxan | - |
| 63 | H | 4-Hydroxyphenyl | H | H | H | H | 186 bis 187 | 0,20 /A/ | D | 80 | Äthanol | - | Dioxan und Methanol im Volumverhältnis von 1 : 1 | - |
| 64 | H | n-Propyl | H | H | H | H | 97 bis 98 | 0,30 /A/ | D | 80 | Gemisch von Äthanol und n-Hexan im Volumverhältnis von 1 : 3 | - | - | - |

Fortsetzung der Tabelle 2

| Verbindung | | Symbole | | | | | Schmelzpunkt in °C | $R_f$-Wert | Verfahrensweise | Ausbeute in % der Theorie | Umkristallisationslösungsmittel | $\alpha_D^{20}$-Wert | Anmerkung | |
|---|---|---|---|---|---|---|---|---|---|---|---|---|---|---|
| Beispiel Nr. | $R_2$ | $R_3$ | $R_3'$ | $R_4$ | $R_6$ | $R_6'$ | | | | | | | Reaktionsmedium abweichend | Katalysator beziehungsweise Base |
| 65 | H | 4-Dimethylaminophenyl | H | H | H | H | 146 bis 147 | 0,70 /E/ | D | 30 | Äthanol | - | Methanol und Dimethylformamid im Volumverhältnis von 1 : 1 | - |
| 66 | H | 4-Carboxyphenyl | H | H | H | H | 170 | 0,20 /D/ | D | 60 | - | - | Methanol und Dimethylformamid im Volumverhältnis von 1 : 1 | - |
| 67 | H | Phenyl | H | H | DL-Äthyl | H | 107 bis 110 | 0,40 /A/ | D | 77 | - | - | - | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 68 | H | 3,4,5-Trimethoxyphenyl | H | H | H | H | 150 bis 151 | 0,20 /A/ | D | 72 | Äthanol | - | - | - |
| 69 | H | Phenyl | H | H | Phenyl | H | 143 bis 144 | 0,50 /A/ | D | 70 | Äthanol | - | Äthylacetat und Dimethylformamid im Volumverhältnis von 1 : 1 | - |
| 70 | H | 2,5-Dimethoxyphenyl | H | H | Phenyl | H | 158 bis 160 | 0,6 /A/ | D | 91 | - | - | Äthylacetat und Dimethylformamid im Volumverhältnis von 1 : 1 | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Anmerkung: Katalysator beziehungsweise Base |
|---|---|---|---|---|---|---|---|---|---|---|---|---|---|---|
| 71 | H | Phenyl | H | H | Phenyl | Phenyl | 184 bis 185 | 0,6 /A/ | D | 64 | Äthanol | - | Dioxan | - |
| 72 | H | Pentamethylen | | H | H | H | 172 bis 173 | 0,4 /A/ | D | 68 | Äthylacetat | - | Äthylacetat und Dimethylformamid im Volumverhältnis von 1 : 1 | - |
| 73 | Acetyl | n-Propyl | H | H | H | H | 102 bis 103 | 0,5 /D/ | G | 56 | Gemisch von Chloroform und n-Hexan im Volumverhältnis von 1 : 3 | - | - | Pyridin b) |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung Reaktionsmedium abweichend | Katalysator beziehungsweise Base |
|---|---|---|---|---|---|---|---|---|---|---|---|---|---|---|
| 74 | Acetyl | 3,4,5-Trimethoxyphenyl | H | H | H | H | 195 bis 196 | 0,4 /A/ | G | 88 | Methanol | - | - | Pyridin b) |
| 75 | Acetyl | Phenyl | H | H | DL-Äthyl | H | 144 bis 145 | 0,5 /A/ | G | 96 | Äthylacetat | - | - | Pyridin b) |
| 76 | Acetyl | Phenyl | H | H | Phenyl | H | 176 bis 177 | 0,3 /D/ | G | 98 | Äthylacetat | - | - | Pyridin b) |

Fortsetzung der Tabelle 2

| Verbindung: Beispiel Nr. | Symbole: $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Anmerkung: Katalysator beziehungsweise Base |
|---|---|---|---|---|---|---|---|---|---|---|---|---|---|---|
| 77 | Acetyl | 4-Hydroxyphenyl | H | H | H | H | 204 | 0,2 /D/ | E | 58 | Äthanol | - | - | - |
| 78 | Acetyl | 4-Dimethylaminophenyl | H | H | H | H | 165 bis 167 | 0,6 /E/ | E | 85 | - | - | - | - |
| 79 | Monochloracetyl | Phenyl | H | H | H | H | 170 bis 171 | 0,5 /D/ | G | 84 | - | - | - | Pyridin b) |
| 80 | Dichloracetyl | Phenyl | H | H | H | H | 170 bis 173 | 0,6 /D/ | G | 80 | - | - | - | Pyridin b) |

Fortsetzung der Tabelle 2

| Verbindung: Beispiel Nr. | Symbole: $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Katalysator beziehungsweise Base |
|---|---|---|---|---|---|---|---|---|---|---|---|---|---|---|
| 81 | Trichloracetyl | Phenyl | H | H | H | H | 188 bis 190 | 0,6 /D/ | G | 69 | - | - | - | Pyridin b) |
| 82 | Trifluoracetyl | Phenyl | H | H | H | H | 139 bis 140 | 0,6 /D/ | E | 75 | - | - | Tetrahydrofuran | - |
| 83 | Acetyl | Pentamethylen | | H | H | H | 159 bis 160 | 0,6 /A/ | E | 71 | - | - | - | - |
| 84 | Acetyl | 4-Carboxyphenyl | H | H | H | H | 232 bis 233 | 0,3 /D/ | E | 40 | Wasser | - | - | - |

Fortsetzung der Tabelle 2

| Verbindung | | | | | | | Schmelzpunkt in °C | $R_f$-Wert | Verfahrensweise | Ausbeute in % der Theorie | Umkristallisationslösungsmittel | $\alpha_D^{20}$-Wert | Anmerkung | |
|---|---|---|---|---|---|---|---|---|---|---|---|---|---|---|
| Beispiel Nr. | Symbole | | | | | | | | | | | | Reaktionsmedium abweichend | Katalysator beziehungsweise Base |
| | $R_2$ | $R_3$ | $R_3'$ | $R_4$ | $R_6$ | $R_6'$ | | | | | | | | |
| 85 | Valeryl | Phenyl | H | H | H | H | 56 bis 58 | 0,5 /D/ | G | 60 | - | - | - | Pyridin b) |
| 86 | Trimethylacetyl | Phenyl | H | H | H | H | 94 bis 97 | 0,6 /D/ | G | 80 | - | - | - | Pyridin b) |
| 87 | Isobutyryl | Phenyl | H | H | H | H | 125 bis 126 | 0,5 /D/ | G | 45 | - | - | - | Pyridin b) |
| 88 | Lauroyl | Phenyl | H | H | H | H | 103 bis 104 | 0,6 /D/ | G | 85 | - | - | - | Pyridin b) |

Fortsetzung der Tabelle 2

| Verbindung: Beispiel Nr. | Symbole: $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Katalysator beziehungsweise Base |
|---|---|---|---|---|---|---|---|---|---|---|---|---|---|---|
| 89 | Äthoxycarbonyl | Phenyl | H | H | H | H | 125 bis 126 | 0,4 /D/ | G | 78 | - | - | - | Pyridin b) |
| 90 | Phenoxycarbonyl | Phenyl | H | H | H | H | 142 bis 143 | 0,7 /A/ | G | 44 | - | - | - | - |
| 91 | 4-Trifluormethylbenzoyl | Phenyl | H | H | H | H | 113 bis 114 | 0,5 /A/ | G | 65 | - | - | Chloroform | wäßrige gesättigte Natriumbicarbonatlösung d) |
| 92 | Carbamoyl | Phenyl | H | H | H | H | 210 bis 211 | 0,2 /A/ | H | 70 | - | - | - | - |

Fortsetzung der Tabelle 2

| Verbindung Beispiel Nr. | Symbole $R_2$ | $R_3$ | $R_3'$ | $R_4$ | $R_6$ | $R_6'$ | Schmelzpunkt in °C | $R_f$-Wert | Verfahrensweise | Ausbeute in % der Theorie | Umkristallisationslösungsmittel | $\alpha_D^{20}$-Wert | Anmerkung: Reaktionsmedium abweichend | Katalysator beziehungsweise Base |
|---|---|---|---|---|---|---|---|---|---|---|---|---|---|---|
| 93 | n-Propylcarbamoyl | Phenyl | H | H | H | H | 198 bis 199 | 0,4 /A/ | H | 44 | - | - | Tetrahydrofuran | Triäthylamin c) |
| 94 | n-Octylcarbamoyl | n-Propyl | H | H | H | H | 104 bis 105 | 0,5 /B/ | H | 43 | - | - | - | Triäthylamin c) |
| 95 | Diäthylaminoäthylcarbamoyl | Phenyl | H | H | H | H | 127 bis 128 | 0,2 /F/ | H | 68 | Gemisch von Äthylacetat und n-Hexan im Volumverhältnis von 1 : 3 | - | - | - |

Fortsetzung der Tabelle 2

| Verbindung Bei-spiel Nr. | Symbole $R_2$ | $R_3$ | $R_3'$ | $R_4$ | $R_6$ | $R_6'$ | Schmelz-punkt in °C | $R_f$-Wert | Ver-fah-rens-weise | Aus-beute in % der Theorie | Umkristalli-sations-lösungs-mittel | $\alpha_D^{20}$-Wert | Anmerkung Reaktions-medium abweichend | Katalysator beziehungsweise Base |
|---|---|---|---|---|---|---|---|---|---|---|---|---|---|---|
| 96 | Benzoyl | n-Propyl | H | H | H | H | 115 bis 117 | 0,5 /A/ | G | 73 | - | - | Chloro-form | wäßrige gesättigte Natriumbicarbonatlösung d) |
| 97 | 4-Chlor-ben-zoyl | Phenyl | H | H | H | H | 125 bis 128 | 0,5 /A/ | G | 79 | - | - | Chloro-form | wäßrige gesättigte Natriumbicarbonatlösung d) |
| 98 | 3,4,6-Trimethoxy-benzoyl | Phenyl | H | H | H | H | 154 | 0,6 /A/ | G | 74 | Methanol | - | - | Pyridin b) |

Fortsetzung der Tabelle 2

| Verbindung | | Symbole | | | | | Schmelzpunkt in °C | $R_f$-Wert | Verfahrensweise | Ausbeute in % der Theorie | Umkristallisationslösungsmittel | $\alpha_D^{20}$-Wert | Anmerkung | |
|---|---|---|---|---|---|---|---|---|---|---|---|---|---|---|
| Beispiel Nr. | $R_2$ | $R_3$ | $R_3'$ | $R_4$ | $R_6$ | $R_6'$ | | | | | | | Reaktionsmedium abweichend | Katalysator beziehungsweise Base |
| 99 | N-(Benzyloxycarbonyl)-glycyl | Phenyl | H | H | H | H | 178 bis 180 | 0,6 /A/ | G | 20 | Methanol | - | - | Pyridin b) |

a) (etwa 100 Mol-% $H_2SO_4$, bezogen auf die Ausgangssubstanz der Formel II)

b) (etwa 110 Mol-%, bezogen auf die Ausgangssubstanz der Formel I)

c) (etwa 100 Mol-%, bezogen auf die Ausgangssubstanz der Formel I)

d) (etwa 300 Mol-% $NaHCO_3$, bezogen auf die Ausgangssubstanz der Formel I)

Patentansprüche

## Patentansprüche

1.) Tetrahydro-1,2,4-oxadiazin-5-onderivate der allgemeinen Formel

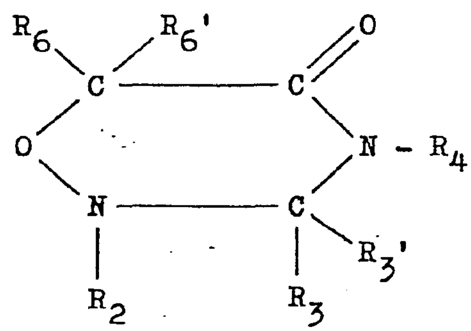

I ,

worin

$R_2$ für Wasserstoff, einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest, einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest oder einen N-(Benzyloxycarbonyl)-glycylrest steht,

$R_3$ einen Alkylrest mit 1 bis 5 Kohlenstoffatom(en), einen, gegebenenfalls durch 1 oder mehr Nitrogruppe(n), Carboxygruppe(n), Hydroxygruppe(n), Dialkylaminorest(e) mit 1 bis 4 Kohlenstoffatom(en) in jedem Alkylteil und/oder Alkoxyrest(e) mit 1 bis 4 Kohlenstoffatom(en) substituierten, Phenylrest, einen Naphthylrest, einen Nitrofurylrest oder einen Thienylrest bedeutet und

$R_3'$ für Wasserstoff steht oder

$R_3$ und $R_3'$ zusammen einen Pentamethylenrest darstellen,

$R_4$ für Wasserstoff oder einen Acetylrest steht,

$R_6$ Wasserstoff, einen Alkylrest mit 1 bis 5 Kohlenstoffatom(en), einen Phenylrest oder einen Benzylrest bedeutet und

$R_6'$ für Wasserstoff steht oder

$R_6$ und $R_6'$ je einen Phenylrest bedeuten,

sowie ihre optisch aktiven Antipoden, und Gemische derselben, im Falle daß $R_6'$ für Wasserstoff steht und $R_6$ einen Alkylrest mit 1 bis 5 Kohlenstoffatom(en), einen Phenylrest oder einen Benzylrest bedeutet.

2.) Tetrahydro-1,2,4-oxadiazin-5-onderivate nach Anspruch 1, dadurch gekennzeichnet, daß das beziehungsweise die Halogenatom(e), durch welches beziehungsweise

welche der Alkylcarbonylrest oder der Benzoylrest, für den $R_2$ stehen kann, substituiert sein kann, Chlor und/oder Fluor ist beziehungsweise sind.

3.) Tetrahydro-1,2,4-oxadiazin-5-onderivate nach Anspruch 1 oder 2, dadurch gekennzeichnet, daß der Alkylcarbonylrest, für den $R_2$ stehen kann, ein solcher mit 1 bis 4, insbesondere 1 oder 2 Kohlenstoffatom(en) im Alkylteil ist.

4.) Tetrahydro-1,2,4-oxadiazin-5-onderivate nach Anspruch 1, dadurch gekennzeichnet, daß der beziehungsweise die Alkylrest(e), durch welchen beziehungsweise welche der Carbamoylrest, für den $R_2$ stehen kann, N-substituiert sein kann, ein solcher beziehungsweise solche mit 1 bis 4, insbesondere 3 oder 4, Kohlenstoffatom(en) ist beziehungsweise sind.

5.) Tetrahydro-1,2,4-oxadiazin-5-onderivate nach Anspruch 1, dadurch gekennzeichnet, daß der beziehungsweise die Dialkylaminoalkylrest(e), durch welchen beziehungsweise welche der Carbamoylrest, für den $R_2$ stehen kann, N-substituiert sein kann, ein solcher beziehungsweise solche mit 1 oder 2, insbesondere 2, Kohlenstoffatom(en) in jedem Alkylteil ist beziehungsweise sind.

6.) Tetrahydro-1,2,4-oxadiazin-5-onderivate nach Anspruch 1 bis 5, dadurch gekennzeichnet, daß der Alkylrest, für den $R_3$ stehen kann, ein solcher mit 1 bis 4, insbesondere 2 oder 3 Kohlenstoffatom(en) ist.

7.) Tetrahydro-1,2,4-oxadiazin-5-onderivate nach Anspruch 1 bis 5, dadurch gekennzeichnet, daß der beziehungsweise die Dialkylaminorest(e), durch welchen beziehungsweise welche der Phenylrest, für den $R_3$ stehen kann, substituiert sein kann, ein solcher beziehungsweise solche mit 1 oder 2, insbesondere 1, Kohlenstoffatom(en) in jedem Alkylteil ist beziehungsweise sind.

8.) Tetrahydro-1,2,4-oxadiazin-5-onderivate nach Anspruch 1 bis 5 oder 7, dadurch gekennzeichnet, daß der beziehungsweise die Alkoxyrest(e), durch welchen beziehungsweise welche der Phenylrest, für den $R_3$ stehen kann, substituiert sein kann, ein solcher mit 1 oder 2, insbesondere 1, Kohlenstoffatom(en) ist beziehungsweise sind.

9.) Tetrahydro-1,2,4-oxadiazin-5-onderivate nach Anspruch 1 bis 8, dadurch gekennzeichnet, daß der Alkylrest, für den $R_6$ stehen kann, ein solcher mit 1 oder 2, insbesondere 1, Kohlenstoffatom(en) ist.

10.) Tetrahydro-1,2,4-oxadiazin-5-onderivate nach Anspruch 1 oder 6 bis 9, dadurch gekennzeichnet, daß der beziehungsweise die Alkoxyrest(e), durch welchen beziehungsweise welche der Benzoylrest, für den $R_2$ stehen kann, substituiert sein kann, ein Methoxyrest beziehungsweise Methoxyreste ist beziehungsweise sind.

11.) 2-Acetyl-3-phenyltetrahydro-1,2,4-oxadiazin-5-on.

12.) 2-Acetyl-3-phenyl-6-methyltetrahydro-1,2,4-oxadiazin-5-on und seine optisch aktiven Antipoden.

13.) 2,4-Diacetyl-3-phenyltetrahydro-1,3,4-oxadiazin-5-on.

14.) 2-Acetyl-3-(4'-fluorphenyl)-tetrahydro-1,2,4-oxadiazin-5-on.

15.) 2-Benzyloxycarbonyl-3-phenyltetrahydro-1,2,4-oxadiazin-5-on.

16.) Verfahren zur Herstellung der Verbindungen nach Anspruch 1 bis 15, dadurch gekennzeichnet, daß man

a) zur Herstellung von Tetrahydro-1,2,4-oxadiazin-5-onderivaten der allgemeinen Formel I, bei welchen

$R_2$ von Wasserstoff verschieden ist und

$R_4$ für Wasserstoff steht,

α-Aminooxycarbonsäureamide der allgemeinen Formel

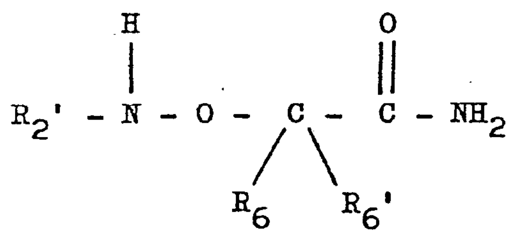

II ,

worin

$R_2'$ für einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest, einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4

Kohlenstoffatom(en) in jedem Alkylteil N-substituierten, Carbamoylrest oder einen N-(Benzyloxycarbonyl)-glycylrest steht

und

$R_6$ und $R_6$' wie im Anspruch 1 oder 9 festgelegt sind,

in protonischen oder aprotonischen Medien in Gegenwart von Säuren mit Oxoverbindungen der allgemeinen Formel

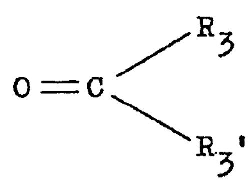

III ,

worin $R_3$ und $R_3$' wie im Anspruch 1 oder 6 bis 8 festgelegt sind, kondensiert

oder

b) zur Herstellung von Tetrahydro-1,2,4-oxadiazin-5-onderivaten der allgemeinen Formel I, bei welchen $R_2$ und $R_4$ Wasserstoff bedeuten, Tetrahydro-1,2,4-oxadiazin-5-onderivate der allgemeinen Formel I,

bei welchen

$R_2$ für einen Benzyloxycarbonylrest steht und

$R_4$ Wasserstoff bedeutet,

in Gegenwart von Katalysatoren mit Wasserstoff hydriert

und/oder

c) zur Herstellung von Tetrahydro-1,2,4-oxadiazin-5-onderivaten der allgemeinen Formel I, bei welchen $R_2$ für einen, gegebenenfalls durch 1 oder mehr Halogenatom(e) substituierten, Alkylcarbonylrest mit 1 bis 12 Kohlenstoffatom(en) im Alkylteil, einen Formylrest, einen, gegebenenfalls durch 1 oder mehr Alkoxyrest(e) mit 1 oder 2 Kohlenstoffatom(en), Halogenatom(e) oder Trifluormethylrest(e) substituierten, Benzoylrest, einen Äthoxycarbonylrest, einen Phenoxycarbonylrest, einen Benzyloxycarbonylrest, einen, gegebenenfalls durch 1 oder 2 Alkylrest(e) mit 1 bis 8 Kohlenstoffatom(en) oder Dialkylaminoalkylrest(e) mit 1 bis 4 Kohlenstoffatom(en) in jedem Alkylteil N-substituierten, Carbamoylrest oder einen N-(Benzyloxycarbonyl)-glycylrest steht, Tetrahydro-1,2,4-oxadiazin-5-onderivate der allgemeinen Formel I, bei welchen $R_2$ für Wasserstoff steht, mit Acylhalogeniden beziehungsweise Carbonsäureanhydriden der allgemeinen Formel

$$R_2' - X \qquad IV ,$$

worin

$R_2'$ wie oben festgelegt ist und

X für ein Halogenatom oder einen Acyloxyrest der allgemeinen Formel

$R_2' - O -$ V ,

in welchletzterer

$R_2'$ wie oben festgelegt ist,

steht,

umsetzt

oder

d) zur Herstellung von Tetrahydro-1,2,4-oxadiazin-5-onderivaten der allgemeinen Formel I, bei welchen $R_2$ einen Formylrest bedeutet, Tetrahydro-1,2,4-oxadiazin-5-onderivate der allgemeinen Formel I, bei welchen $R_2$ für Wasserstoff steht, in Gegenwart von Kondensationsmitteln mit Ameisensäure umsetzt

oder

e) zur Herstellung von Tetrahydro-1,2,4-oxadiazin-5-onderivaten der allgemeinen Formel I, bei welchen $R_4$ für einen Acetylrest steht, Tetrahydro-1,2,4-oxadiazin-5-onderivate der allgemeinen Formel I, bei welchen $R_4$ Wasserstoff bedeutet, unter energischen Reaktionsbedingungen mit Acetylchlorid oder Essigsäureanhydrid umsetzt

oder

f) zur Herstellung von Tetrahydro-1,2,4-oxadiazin-5-onderivaten der allgemeinen Formel I, bei welchen $R_2$ für einen, gegebenenfalls durch 1 oder 2 Alkylrest(e) mit 1 bis 8 Kohlenstoffatom(en) oder Dialkylaminoalkylrest(e) mit

1 bis 4 Kohlenstoffatom(en) in jedem Alkylteil N-substituierten, Carbamoylrest steht, Tetrahydro-1,2,4-oxadiazin-5-onderivate der allgemeinen Formel I, bei welchen $R_2$ einen Phenoxycarbonylrest bedeutet, mit Ammoniak oder mit passenden primären oder sekundären Aminen umsetzt

oder

g) zur Herstellung von Tetrahydro-1,2,4-oxadiazin-5-onderivaten der allgemeinen Formel I, bei welchen $R_2$ für einen durch einen Alkylrest mit 1 bis 8 Kohlenstoffatom(en) N-substituierten Carbamoylrest steht, Tetrahydro-1,2,4-oxadiazin-5-onderivate der allgemeinen Formel I, bei welchen $R_2$ Wasserstoff bedeutet, mit Alkylisocyanaten der allgemeinen Formel

$$R - N{=}C{=}O \qquad VI ,$$

worin R für einen Alkylrest mit 1 bis 8 Kohlenstoffatom(en) steht, umsetzt.

17.) Arzneimittel, gekennzeichnet durch einen Gehalt an 1 oder mehr Verbindung(en) nach Anspruch 1 bis 14 als Wirkstoff(en), gegebenenfalls zusammen mit 1 oder mehr pharmazeutisch üblichen festen oder flüssigen Trägerstoff(en) und/oder Hilfsstoff(en).

Zusammenfassung

Europäisches Patentamt

# EUROPÄISCHER RECHERCHENBERICHT

0055490

Nummer der Anmeldung

EP 81 11 0829.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D,A | TETRAHEDRON, Band 35, Nr. 11, 1979<br>Oxford, New York, Toronto<br>F.G. RIDDELLet al. "The Synthesis and Conformational Analysis of Tetrahydro-1,2,4-oxadiazines"<br>Seiten 1391 bis 1398<br>* Seite 1392, Schema *<br>-- | 16 | C 07 D 273/04<br>C 07 D 413/04<br>A 61 K 31/535 |
| A | DE - A1 - 2 640 464 (PHILAGRO S.A.)<br>---- | | RECHERCHIERTE SACHGEBIETE (Int. Cl.³)<br>A 61 K 31/535<br>C 07 D 273/04 |
| | | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 16-03-1982 | FROELICH |